# EUROPEAN PATENT APPLICATION

(11) **EP 4 760 731 A1**
(43) Date of publication of application: **17.06.2026**
(21) Application number: 24383361.3
(22) Date of filing: 12.12.2024
(51) Int. Cl.: G16H 10/40, G01N 35/00, G16H 40/20

(54) **WORKFLOW PLANNING MANAGER**

(71) Applicant: Roche Diagnostics International AG, 6343 Rotkreuz (CH)
(72) Inventor: FERNANDEZ GULIAS, Carlos, 6343 Rotkreuz (CH); NADAL ROIG, Esteve, 6343 Rotkreuz (CH); JIMINEZ RODA, Javier, 6343 Rotkreuz (CH); WEILEMANN, Mathias Markus, 6343 Rotkreuz (CH); VON HOPFFGARTEN, Moritz, 6343 Rotkreuz (CH); TANNER, Stefan Andreas, 6343 Rotkreuz (CH)
(74) Representative: Mewburn Ellis LLP

(57) **Abstract**

A workflow planning manager for coordinating the distribution of biological samples in an analytical system is provided. The analytical system comprises: one or more diagnostic analyzers for performing one or more tests on the biological samples, and a transport device for transporting the biological samples to the one or more diagnostic analyzers. The workflow planning manager is configured to: receive a plurality of requests for analytical tests to be processed by the system, each analytical test corresponding to a biological sample; determine a proposed run comprising at least some of the requested analytical tests; transmit the proposed run to a candidate diagnostic analyzer of the one or more diagnostic analyzers, receive predicted run fulfilment information from the candidate diagnostic analyzer, the predicted run fulfilment information comprising an indication of which of the requested analytical tests in the proposed run can be processed by the candidate diagnostic analyzer; and determine, whether to proceed with or to reject the proposed run based on the predicted run fulfilment information. When the proposed run is to be proceeded with, the workflow planning manager is configured to signal the transport device to transport some or all of the biological samples specified in the proposed run to the candidate diagnostic analyzer, and when the proposed run is rejected, the workflow planning manager is configured to determine an alternative proposed run or transmit the proposed run to an alternative candidate diagnostic analyzer.

## Description

### Field of the Invention

The present invention relates to a workflow planning manager for coordinating the distribution of biological samples in an analytical system; and a computer-implemented method for coordinating an analytical system.

### Background

Laboratory environments, such as a molecular laboratories, may contain automated laboratory equipment, such as IVD (in vitro diagnostic) instruments, which are used to perform various processes and tests on biological samples. Typically, a planning manager of the laboratory is configured to receive one or more test orders describing which processes to carry out on each biological sample and direct the laboratory equipment to carry out the test orders.

To process each test order, numerous resources may be required including instrument resources (e.g., transport systems for sample holders and reaction vessels, robotic arms with pipettes to transfer and dose samples and/or reagent liquids, fluid mixers, incubators, photodetectors, etc) and consumable resources (e.g., reagents, disposable fluid carriers such as tubes, reaction vessels, multi-well plates or other fluid carriers for sample fluids and reagents, disposable pipette tips, etc). The sequence of using these resources to process a test order can be complex and typically includes pre-analytical, analytical and post-analytical steps. Furthermore, multiple processes involved in processing a test order may be run fully or partially in parallel and share some of the same resources thus introducing added complexity.

Moreover, biological samples and test orders corresponding to each biological sample may arrive in the laboratory in a continuous flow during working hours, each of which must be scheduled and processed. Each test order and biological sample may have different characteristics including: different types of test to be performed (e.g., HBV, HIV, etc), and target Turnaround Times (TAT) for processing each test. Further, each test order may be classified as an urgent or a routine test order. Multiple samples may be processed in parallel by the laboratory equipment to increase throughput. However, incompatibilities between tests exist which means that two incompatible tests cannot be processed at the same time, e.g., in a same run.

A series of test orders to be performed by an instrument (e.g., a diagnostic analyzer such as an IVD instrument) may be referred to as a run. It is desirable to plan a run so that samples are loaded into each of the diagnostic analyzers in an order and combination which accommodates and optimizes factors such as priorities of the test orders, material usage, target TATs, cost, sample transport time, and waste generation. Accordingly, the development and operation of an efficient workflow for the automated laboratory equipment is challenging.

Existing solutions for coordinating a laboratory typically employ simple algorithms for ordering samples and processes which are not flexible or adaptable (e.g., to newly incumbent test orders or sample transport errors). Additionally, existing solutions are ineffective for defining laboratory-wide workflows forcing users to pre-sort biological samples before loading them into the laboratory equipment.

Further, such existing solutions typically implement top-down, long-term planning schemes whereby sample workflows are determined based on prior knowledge of incoming test orders. In these examples, the laboratory equipment is provided with a predefined list of tests to process. This list is typically the result of a tactical decision made in advance, e.g., daily or weekly. However, such solutions require central control and up-to-date knowledge of the resources that are available in each instrument of the laboratory. Further, these solutions are ineffective at reacting to changes in the system such as incoming test orders, sample identifier errors, bottlenecks in sample transportation systems, changing states of the laboratory equipment, and changing resources levels. Moreover, such solutions experience difficulty predicting which samples are going to arrive in the laboratory and when, which makes it difficult to accurately plan throughout the day. Additionally, unfilled runs of test orders maybe scheduled (e.g., if urgent samples are received) which decreases the overall occupation and efficiency of the system.

Therefore, there is a desire for a more effective and adaptable method for managing the distribution of samples and test orders in an analytical system. The present invention has been devised in light of the above considerations.

### Summary of the Invention

Broadly, the present invention relates to a system for coordinating diagnostic analyzers wherein a planning manager proposes a run of tests to be performed by one of the diagnostic analyzers. The proposed run is transmitted to one of the diagnostic analyzers to assess how much of the proposed run the diagnostic analyzer can fulfil based on current resource levels and capabilities of that analyzer. The planning manager can then adjust the proposed run based on that information until a run with an acceptable level of fulfilment is arrived at. Further, as changes such as new samples or test orders arriving in the system or changes in resource statuses are detected, the planning manager can renegotiate the proposed run in order to accommodate the detected changes.

Accordingly, in a first aspect, embodiments of the present invention provide: a workflow planning manager for coordinating the distribution of biological samples in an analytical system. The analytical system comprises: one or more diagnostic analyzers for performing one or more tests on the biological samples, and a transport device for transporting the biological samples to the one or more diagnostic analyzers. The workflow planning manager is configured to: receive a plurality of requests for analytical tests to be processed by the system, each analytical test corresponding to a biological sample, determine a proposed run comprising at least some of the requested analytical tests; transmit the proposed run to a candidate diagnostic analyzer of the one or more diagnostic analyzers, receive predicted run fulfilment information from the candidate diagnostic analyzer, the predicted run fulfilment information comprising an indication of which of the requested analytical tests in the proposed run can be processed by the candidate diagnostic analyzer, and determine, whether to proceed with or to reject the proposed run based on the predicted run fulfilment information. When the proposed run is to be proceeded with, the workflow planning manager is configured to signal the transport device to transport (e.g., some or all of) the biological samples specified in the proposed run to the candidate diagnostic analyzer. When the proposed run is rejected, the workflow planning manager may be configured to determine an alternative proposed run or transmit the proposed run to an alternative candidate diagnostic analyzer.

Advantageously, the workflow planning manager is able to react to changing resources and incoming test orders in the system by planning the distribution of samples on a run-by-run basis. Further, by transmitting the proposed run to the analyzer for assessment, the workflow planning manager is able to use the most up-to-date information on the analyzers' capabilities without needing to control the analyzers themselves. In this way, the workflow planning manager is able to ensure that optimal laboratory workflows can be determined and updated based on evolving information so as to minimise resource usage and maximise laboratory throughput whilst ensuring that target TAT's and urgency statuses are complied with.

Further, by negotiating with the candidate diagnostic analyzer in this way, rather than requesting information on the candidate analyzers' current resources and capabilities, Demeter's law [1] is followed whereby the processing is separated between components of the system, thus limiting the amount of coupling between the components. As a result, the analytical system is more reactive and adaptable to changes in the system (such as the removal or addition of diagnostic analyzers or updates to the existing analyzers) than existing analytical systems.

The workflow planning manager (e.g., coordinator, planning module, etc) may be a central processing component of the system such as a server or processor which is communicatively coupled to each of the diagnostic analyzers and the transport device.

The analytical system may be an automated laboratory such as a molecular laboratory. The automated laboratory may include a plurality of diagnostic analyzers (e.g., instruments, analyzers, IVD instruments, analytical devices, etc) configured to perform pre-analytical, analytical and/or post-analytical steps of tests on each of the biological samples.

The requests for analytical tests may be test orders, each test order being associated with one or more of the biological samples. For example, each test order may specify: one or more processing steps to perform on the associated biological sample(s), a target turn-around time (TAT) within which the test order should be processed, and/or an urgency status indicating whether the test order is an urgent or routine test orders. Urgent test orders may be considered to be high priority test orders which should be processed without delay.

The requests for analytical tests (e.g., test orders) may be received from third party systems such as healthcare centres, research centres, hospitals, doctors' surgeries, etc and/or from a user inputting the test orders to an interface device. Each analytical test may indicate an identifier (ID) corresponding to one of the biological samples. Each biological sample may be identified (when it arrives at and/or when it is within the analytical system) by an ID reader, such as a barcode reader or a QR code reader, which is configured to read an ID attached to a sample container which houses the biological sample.

A run may refer to a list or schedule of analytical tests to be processed by a diagnostic analyzer. One or more of the tests specified in each run may be executed by the diagnostic analyzer in parallel or partially in parallel. The biological samples specified by the requested tests in a run may be transported within one or more racks to the candidate analyzer. Each run may correspond to (e.g., comprise) one or more racks of biological samples within the analytical system. For example, each run may include between 20 to 200, more preferably, 50 to 150, more preferably 96 tests (and/or biological samples). Each rack may comprise a corresponding number of slots for housing biological samples in sample containers.

The transportation of some or all of the biological samples in the proposed run may include the transportation of all of the biological samples corresponding to the analytical tests which are specified in the proposed run (irrespective of whether those tests are indicated in the predicted run fulfilment information or not). In this way, the racks which are used for transporting the biological samples need not be rearranged or re-ordered before transportation. In other examples, the transportation of some or all of the biological samples in the proposed run may include transportation of (only) the biological samples corresponding to the analytical tests which are specified in the proposed run, and which are also identified in the predicted run fulfilment information. Therefore, only the biological samples which are expected to be executed by diagnostic analyzer may be transported to the diagnostic analyzer.

The workflow planning manager may be configured to receive the predicted run fulfilment information from the candidate diagnostic analyzer via an analyzer interface module. The analyzer interface module, discussed in detail below, may be configured to provide an interface for communications between the workflow planning module and one or more of the diagnostic analyzers.

Determining the proposed run may comprise: selecting a plurality of tests from the requested tests according to a workflow optimisation scheme, and populating the proposed run with the selected tests. The workflow optimisation scheme may include one or more optimisation goals or factors which the workflow planning manager is configured to optimise in constructing the proposed run.

For example, selecting the plurality of tests according to the workflow optimisation scheme may comprise selecting analytical tests, from the requested tests, based on one or more of the optimization factors. For example, analytical tests may be selected based on any one or more of: a number of tests per type of test in the requested tests, a target turn-around-time of each requested test, compatibilities between the requested tests (e.g., for processing by a same diagnostical analyzer), total TAT (to process all of the selected analytical tests), individual sample TAT (of each analytical test), QC (quality control) material use needed to perform each test, reagent usage (or other resources), diagnostic analyzer load balancing, minimisation of cost and/or resource usage, and/or filling of runs. Such factors may be considered so as to select requested test for the proposed run which: are able to be processed in a same diagnostic analyzer, have compatible target turn-around-times, and/or are tests of a same type. By constructing a run according to one or more of these factors, the time taken to execute the run can be reduces whilst minimising overall resource usage of the analytical system.

In some examples, selecting the plurality of tests according to the workflow optimisation scheme may comprise: identifying requested tests for which an associated target TAT has already elapsed. Further, the requested tests for which the target TAT has been elapsed for the greatest period of time may then be prioritised for populating the proposed run (e.g., such requested tests may be selected for a proposed run first, ahead of other requests tests for which the target TAT falls later).

Further, in some examples, selecting the plurality of tests according to the workflow optimisation scheme may comprise minimising a number of passenger samples in the proposed run. Passenger samples may be understood to mean biological samples that are included in a rack which has been sent to one of the analyzers for execution of a run, but which correspond to requested tests that are not compatible with that analyzer and/or other requested tests in that run. Accordingly, passenger samples may be samples which are transported to an analyzer but are not actually processed in that analyzer. By minimizing the presence of passenger samples in a run, more tests may be performed per run thus increasing the efficiency of the system and reducing a time and cost per test being processed in the system.

In some examples, selecting the plurality of tests according to the workflow optimisation scheme may comprise: selecting requested tests which are indicated as urgent, and/or selecting tests having the soonest target turn-around-times of the requested tests. The optimisation scheme may require that the requested tests which are indicated as urgent are selected first, and then the requested tests having the soonest target turn-around-times are selected.

In some examples, selecting the plurality of tests for the proposed run (according to the workflow optimisation scheme) may comprise a set of : for example, using Mixed-Integer Programming, MIP, based on one or more cost functions and/or optimisation goals or factors (e.g., predicted turn-around-times, target turn-around-times, and/or urgency statuses associated with each of the requested tests) to optimise the proposed run against a workflow performance metric such as number of passenger samples, time overdue, load balancing, resource usage, or any other suitable workflow performance metric.

Selecting the plurality of tests for the proposed run may include any combination of the above described considerations. For example, the selection may account for test urgency and/or target turn-around-times and/or test compatibilities and/or passenger samples etc.

The workflow planning manager may be configured to fill the proposed run with a target fill number of requested tests. For example, the target fill number may be a number of tests required to fill a run (or a rack). For example, the target fill number may be between 20 and 200 tests per run, or more preferably 90 tests per run. The target fill number may be a maximum capacity of a run (e.g., 96 test orders/samples) or the target fill number may be less than the maximum capacity of a run (e.g., between 80% and 95% of the maximum capacity, e.g., 90 test orders or samples). Filling runs in this way, before sending them to an analyzer, can advantageously reduce cost, resource usage and TAT while maximising efficiency of the analytical system as a whole.

Determining the proposed run may comprise: selecting an initial plurality of tests (e.g., according to the workflow optimisation scheme), and then filling the proposed run by allocating additional tests to the proposed run to meet the target fill number. The additional tests may be selected according to a filling scheme which is configured to maximise a utility of each proposed run. Advantageously, by filling each run (e.g., with less urgent tests) the system may be able to operate more efficiently and process more test orders in a shorter amount of time.

Filling the proposed run may comprise allocating requested tests to the proposed run according to an order of target turn-around-times associated with each of the requested tests (e.g., from soonest to latest, shortest to longest etc).

The system may further comprise a buffer (e.g., a storage area or holding rack) for storing the biological samples before transportation to one or more of the diagnostic analyzers. Filling the proposed run may then comprise allocating requested tests to the proposed run according to a time of arrival of the biological samples in the buffer (e.g., from oldest to youngest, from least to most recent, earliest to latest, etc). In this way, the biological samples which arrived in the buffer first may be allocated to a run before biological samples which arrived later thus reducing the average individual TAT of samples in the system.

In further examples, filling the proposed run may comprise allocating requested tests to the proposed run according to a time of receipt of the requested test by the workflow planning manager (e.g., from oldest to youngest, from least to most recent, from earliest received to latest received etc). In this way, older test orders (e.g., received over the internet) may be prioritized even if the biological samples associated with those test orders physically arrived in the analytical system more recently than other biological samples.

The workflow planning manager may be further configured to: determine a maximum delay which can be applied while still meeting target turn-around-times associated with each of the requested tests in the proposed run. The workflow planning manager may then wait for the maximum delay before transmitting the proposed run to the candidate diagnostic analyzer. In this way, a likelihood of the proposed run being filled and of the proposed run being filled with compatible tests is increased since, by waiting for the delay, an increased opportunity is provided for more test orders to arrive in the system, thus triggering the proposed run to be recalculated and potentially improved. As a result, the overall efficiency and throughput of the laboratory can be improved.

The workflow planning manager may be configured to validate the proposed run prior to transmission to the candidate diagnostic analyzer by determining if the proposed run is full and/or if the proposed run is urgent. When the proposed run is determined to be full or urgent, the proposed run may be transmitted to the candidate diagnostic analyzer, and when the proposed run is determined to be neither full nor urgent, the proposed run may be discarded. A proposed run may be determined to be full when a number of requested tests included in the proposed run is equal to or exceeds the aforementioned target fill number, or the maximum capacity of the run.

The workflow planning manager may be further configured to: detect a system change and responsive to detecting the system change, determine the proposed run. The system change may be detected in response to a detection of any one or more of: a new biological sample arriving in the system, a requested test being added or removed from the plurality of requested tests, or an operating status of one of the diagnostic analyzers being updated.

The workflow planning manager may be configured to repeatedly (and continuously) determine a new proposed run in response to detecting a system change until each of the requested tests is executed. Advantageously, by continuously re-determining runs the workflow planning manager is able to adapt the pending workflow and runs to accommodate changes in the system in an efficient manner without being tied to a particular precalculated workflow. For example, urgent samples incumbent in the system may be included in a new run with additional samples, as opposed to the workflow planning manager simply disrupting a predefined and inflexible workflow to process the urgent samples first.

The workflow planning manager may be configured to transmit the proposed run to a plurality of candidate diagnostic analyzers and receive respective predicted run fulfilment information from each candidate diagnostic analyzer. Determining whether to proceed with or reject the proposed run may then comprise analyzing the predicted run fulfilment information from each candidate diagnostic analyzer. Proceeding with the proposed run may then comprise selecting, based on the predicted run fulfilment information, one of the candidate diagnostic analyzers for executing the proposed run.

The workflow planning manager may be configured to select the candidate diagnostic analyzer(s) based on an operating status of each diagnostic analyzer in the system and/or based on an index (e.g., a menu) of analytical tests which are performable by each diagnostic analyzer. In some examples, the workflow planning manager may be configured to select the candidate diagnostic analyzer so as to balance a workload of the diagnostic analyzers. In other examples, the workflow planning manager may be configured to select the candidate diagnostic analyzer by selecting an available analyzer which has been in an idle state the longest.

Determining whether to proceed with or reject the proposed run may be based on the results of an optimization checking process. For example, determining whether to proceed with or reject the proposed run may comprise comparing the predicted run fulfilment information to acceptance criteria (so as to optimise the proposed run according to the acceptance criteria). The acceptance criteria may comprise one or more of: a threshold percentage of requested tests in the proposed run which can be fulfilled by the candidate diagnostic analyzer, whether there are requested tests in the proposed run indicated as urgent, and if those requested tests indicated as urgent can be fulfilled by the candidate diagnostic analyzer, and/or a target time for beginning the proposed run. If one or more of the acceptance criteria is not satisfied by the predicted run fulfilment information, then the workflow planning manager may reject the proposed run.

The predicted run fulfilment information may comprise an estimated turn-around-time to process the proposed run at the candidate diagnostic analyzer. The optimisation checking process for determining whether to proceed with or reject the proposed run may then comprise: determining if the estimated turn-around-time is compatible with associated target turn-around-times of the requested tests in the proposed run. Accordingly, when the estimated turn-around-time is longer or later than the one or more of the target turn-around times, the workflow planning manager may reject the proposed run. When the estimated turn-around-time is shorter or earlier than the one or more of the target turn-around times, the workflow planning manager may proceed with the proposed run.

When the proposed run is proceeded with, the workflow planning manager may be further configured to send a confirmation message to the candidate diagnostic analyzer indicating that the proposed run (and the predicted run fulfilment information) has been accepted. As mentioned above, the transport device may then transport some or all of the biological samples specified in the proposed run to the candidate diagnostic analyzer. This may include transporting the biological samples corresponding to the requested tests which are in the proposed run and which are also indicated in the predicted run fulfilment information to the candidate diagnostic analyzer.

When the proposed run is accepted (e.g., determined to be proceeded with), the workflow planning manager may be configured to receive (or wait to receive) a transport update from the candidate diagnostic analyzer indicating which of the biological samples have arrived at the candidate diagnostic analyzer. The workflow planning manager may then transmit a signal to the diagnostic analyzer indicating, based on the transport update, whether to execute the proposed run, discard the proposed run, modify the proposed run, or delay the proposed run. For example, if all of the biological samples specified in the proposed run (and/or in predicted run fulfilment information) have arrived at the candidate diagnostic analyzer, then the workflow planning manager may signal the candidate diagnostic analyzer to execute the proposed run. If one or more biological samples have not arrived at the candidate diagnostic analyzer, then the workflow planning manager may signal the diagnostic analyzer to execute the proposed run anyway, with the samples it has, provided that tests indicated as urgent can be performed and/or that, at least a predetermined minimum number of the required samples have arrived at the diagnostic analyzer. The workflow planning manager may include test orders associated with the biological samples which have not arrived at the candidate diagnostic analyzer in the determination of a new (subsequent) proposed run so that the unprocessed test orders can be rescheduled.

The workflow planning manager may be further configured to detect an end of shift condition indicating that no further requested tests are in-coming ( e.g., for a current shift, period of time, day etc). Responsive to detecting an end of shift condition, the workflow planning manager may adjust the optimisation checking process to relax (e.g., reduce) the acceptance criteria for determining whether to proceed with or reject the proposed run and/or lower a target fill number for filling the proposed run. For example, when an end of shift condition is detected, the workflow planning manager may be configured to proceed with proposed runs, even if they are not full, so as to finish executing each of the requested tests in that shift.

In a second aspect, embodiments of the present invention provide an analytical system for analyzing biological samples. The analytical system comprises: one or more diagnostic analyzers for performing an analysis on the biological samples, a transport device for transporting the biological samples to the one or more diagnostic analyzers, a workflow planning manager for coordinating the distribution of biological samples to the diagnostic analyzers, the workflow planning manager being communicatively connected to each diagnostic analyzer and to the transport device. The workflow planning manager is configured to: receive a plurality of requests for analytical tests to be processed by the system, each analytical test corresponding to a biological sample, determine a proposed run comprising at least some of the requested analytical tests, transmit the proposed run to a candidate diagnostic analyzer of the one or more diagnostic analyzers, receiving predicted run fulfilment information from the candidate diagnostic analyzer, the predicted run fulfilment information comprising an indication of which of the requested analytical tests in the proposed run can be processed by the candidate diagnostic analyzer, and determining whether to proceed with or reject the proposed run based on the predicted run fulfilment information, wherein: when the proposed run is to be proceeded with, the workflow planning manager is configured to signal the transport device to transport some or all of the biological samples specified in the proposed run to the candidate diagnostic analyzer. When the proposed run is rejected, the workflow planning manager may be configured to: determine an alternative proposed run or transmit the proposed run to an alternative diagnostic analyzer.

The workflow planning manager of the analytical system may include any combination of features described above in relation to the first aspect.

In a third aspect, there is provided a computer-implemented method for coordinating an analytical system for analyzing biological samples, the system comprising: one or more diagnostic analyzers for performing an analysis, each diagnostic analyzer comprising an analyzer programming module for operating the diagnostic analyzer; a transport device for transporting the biological samples to the one or more diagnostic analyzers; and a workflow planning manager for performing the computer-implemented method. The method comprises: receiving a plurality of requests for analytical tests to be processed by the system, each analytical test corresponding to a biological sample, determining a proposed run comprising at least some of the requested analytical tests, transmitting the proposed run to a candidate diagnostic analyzer of the one or more diagnostic analyzers, receiving predicted run fulfilment information from the analyzer programming module of the candidate diagnostic analyzer, the predicted run fulfilment information comprising an indication of which of the requested analytical tests in the proposed run can be pro-cessed by the candidate diagnostic analyzer, determining whether to proceed with or reject the proposed run based on the predicted run fulfilment information, when the proposed run is proceeded with, signal the transport device to transport some or all of the biological samples specified in the proposed run to the candidate diagnostic analyzer, and when the proposed run is rejected, determine an alternative proposed run or transmitting the proposed run to an alternative diagnostic analyzer device.

As mentioned above, the planning module may communicate with the diagnostic analyzer via an analyzer interface module which is configured to manage communications between the planning module and one or more of the diagnostic analyzers. The functions of the analyzer interface module may be referred to as middleware for the analytical system which may act as a bridge between the planning functions of the workflow planning manager and the individual diagnostic analyzers.

Accordingly, in a fourth aspect of the present invention, there is provided: an analyzer interface module for interfacing between a diagnostic analyzer and a workflow planning manager of an analytical system for analyzing biological samples. The analyzer interface module is configured to: receive a proposed run from the workflow planning manager, the proposed run comprising a plurality of requested analytical tests to be executed by the diagnostic analyzer on one or more biological samples, determine a candidate run schedule for executing the requested analytical tests in the proposed run according to an analyzer resource optimisation process, generate predicted run fulfilment information based on the candidate run schedule, the predicted run fulfilment information comprising an indication of which of the requested analytical tests in the proposed run can be processed by the analyzer in the candidate run schedule, and transmit the predicted fulfilment information to the workflow planning manager.

Advantageously, by negotiating a proposed run with the workflow planning manager in this way, the workflow planning manager is able to gain an up-to-date understanding of how much of a proposed run can be fulfilled based on current resource levels and statuses of the analyzer, whilst still operating the processing components of the analytical system in a modular fashion. For example, by running the analyzer resource optimisation process locally, at the analyzer (or in the analyzer interface module) the predicted run fulfilment information can be determined based on the most recent optimization software running on the analyzer, without needing to recreate said optimization software on the workflow planning manager. The analyzer interface module therefore enables diagnostic analyzers in the analytical system to be updated and replaced more easily than in traditional analytical system whilst ensuring efficient operation and throughput of the analytical system.

The diagnostic analyzer may be one of the diagnostic analyzers (e.g., the candidate diagnostic analyzer) referred to above in relation to the first aspect. The interface module may comprise or be formed of middleware for managing communications between the workflow planning module and one or more diagnostic analyzers in an analytical system.

The analyzer interface module may be connected to one or more diagnostic analyzers. For example, the analyzer interface module may be a separate component to the diagnostic analyzer, for example, in the form of software running on a central server and connected to one or more diagnostic analyzers. Alternatively, each analyzer interface module may form part of a diagnostic analyzer, e.g., as a software application running on the diagnostic analyzer. In these examples, the analyzer interface module may be understood to mean an outward facing portion of control software of the diagnostic analyzer which is configured to receive and process communications from the workflow planning manager.

The predicted run fulfilment information may further comprise a predicted total turnaround time (TAT) and/or an available start time of the candidate run schedule. The available start time may be an absolute time or a relative time period indicating when the diagnostic analyzer may be next available to execute the proposed run. The predicted run fulfilment may be considered to be a counteroffer (e.g., counter proposal, modified proposed run etc) for the proposed run, indicating which of, and when, each of the requested tests in the proposed run may be executed by the that diagnostic analyzer. The workflow planning manager may then accept or reject the counteroffer. The proposed run, as modified by the predicted run fulfilment information, may therefore be considered as an agreed run for execution by a diagnostic analyzer.

Determining the candidate run schedule according to the analyzer resource optimisation process may comprise: determining a set of analyzer resources usable (e.g., needed, required, desired) for executing the requested analytical tests in the proposed run. The set of analyzer resources may then be compared to an inventory of available analyzer resources so as to determine which of the requested analytical tests in the proposed run can be executed by that analyzer. The analyzer resource optimisation process may be a local workflow planning process performed locally by one or more of the diagnostic analyzers (or analyzer interface module). Further, the analyzer resource optimisation process may be proprietary software which is configured to determine workflows specifically for the type of diagnostic analyzer which it is installed on.

The inventory of available analyzer resources may comprise one or more of: analyzer hardware, loaded reagent cassettes, loaded control miniracks, and/or time slots indicating periods when the analyzer hardware is available.

The proposed run may comprise a predicted or planned time of arrival of the biological samples at the analyzer. Determining the candidate run schedule according to the analyzer resource optimisation process may then comprise comparing the set of analyzer resources to a predicted inventory of available analyzer resources at the predicted time of arrival (e.g., the analyzer resources predicted to be installed and available in the diagnostic analyzer at the predicted time of arrival of the biological samples).

Responsive to transmitting the predicted run fulfilment information, the analyzer interface module may be further configured to receive a confirmation message from the workflow planning manager indicating that the proposed run and/or the candidate run schedule has been accepted and provide the proposed run to the analyzer for execution according to the candidate run schedule.

The analyzer interface module may be configured to assign an allocation slot (e.g., a reservation sot) of the diagnostic analyzer to the candidate run schedule. The allocation slot may be assigned after (e.g., in response to) receiving the indication that the candidate run schedule has been accepted. The assignment of the allocation slot may by a reservation of that slot thereby prevent the scheduling of further proposed runs in that slot. The diagnostic analyzer may therefore be primed to receive the biological samples and execute the proposed run in the allocation slot which is assigned to that run.

After assigning an allocation slot to the accepted proposed run, the analyzer interface module may be configured to determine a predicted resource availability of consumables and/or of waste storage in the diagnostic analyzer during that allocation slot. If the predicted resource availability is determined to be insufficient to execute the proposed run, then analyzer interface module may send a request to the workflow planning manager for additional resource. The request may be for additional consumable(s) to be provided and/or for the waste storage to be emptied or replaced. For example, if a consumable or waste storage is predicted to be exhausted before a time of the allocation slot assigned to the proposed run, then the resource availability may be determined to be insufficient.

The analyzer interface module may be further configured to reserve analyzer resources, such as consumables, analyzer hardware, etc, which are required (e.g., needed, desired) for executing the accepted proposed run (in the assigned allocation slot).

The analyzer interface module may be configured to receive one or more further proposed runs and determine additional candidate run schedules for each of the further proposed runs. Determining the additional candidate run schedules may comprise accounting for the reserved analyzer resources and/or reserved allocation slots so as to avoid allocating the reserved resources and/or a reserved allocation slot to the further proposed runs.

The analyzer interface module may be configured to receive an alert from the diagnostic analyzer indicating when some or all of the biological samples in the accepted candidate run schedule are received at the analyzer. The analyzer interface may then transmit an indication to the workflow planning manager indicating which of the biological samples have been received at the diagnostic analyzer.

The analyzer interface module may be configured to trigger the diagnostic analyzer to execute the accepted candidate run schedule based on the received biological samples in response to receiving a workflow order from the workflow planning manager.

The analyzer interface module may be configured to cancel execution of a reserved allocation slot and proposed run in response to receiving a cancellation order from the workflow planning manager, and/or in response to determining, at a time of the reserved allocation slot, that expected biological samples needed to execute the proposed run have not arrived at the diagnostic analyzer (and a workflow order has not been received from the workflow planning manager). Cancelling the proposed run and/or the reserved allocation slot may comprise also cancelling any subsequent reserved allocation slots. In this way, the run scheduled for subsequent reserved allocation slots, which may have been determined under the premise that the (first) reserved slot would be realised, can be recalculated based on the most up-to-date information including resources available and remaining unprocessed test orders.

Thus, the interface module enables the system to be more reactive to changes and employ more efficient workflows compared to solutions which require pre-planned runs to remain un-changed.

In a fifth aspect, there is provided a computer-implemented method for an analyzer interface module for interfacing with a diagnostic analyzer and a workflow planning manager of an analytical system for analyzing biological samples, the method comprising: receiving a proposed run from the workflow planning manager, the proposed run comprising a plurality of requested analytical tests to be executed by the diagnostic analyzer on one or more biological samples, determining a candidate run schedule for executing the requested analytical tests in the proposed run according to an analyzer resource optimisation process, generating predicted run fulfilment information based on the candidate run schedule, the predicted run fulfilment information comprising an indication of which of the requested analytical tests in the proposed run can be processed by the analyzer in the candidate run schedule, and transmitting the predicted fulfilment information to the workflow planning manager.

The computer-implemented method may be performed by the analyzer interface module discussed above in any of the preceding aspects.

In further examples, the computer-implemented method may be performed by the analytical system comprising a workflow planning manager and at least one analyzer interface module. Thus, the computer-implemented method may therefore be a distributed computer-implemented method performed by the workflow planning manager and/or the at least one analyzer interface module. The distributed computer-implemented method may therefore comprise any of the features discussed above in respect of the third aspect including steps performed by the workflow planning manager.

In a sixth aspect, there is provided a system for implementing the computer-implemented method of the third and/or fifth aspects.

In a seventh aspect of the present invention, there is provided an analytical system for analyzing biological samples, the system comprising: a diagnostic analyzer for performing an analysis on the biological samples, a transport device for transporting the biological samples to the analyzer, and a workflow planning manager for coordinating the distribution of biological samples in the system, the workflow planning manager being communicatively connected to the analyzer interface module and the transport device, and an analyzer interface module for interfacing between the diagnostic analyzer and the workflow planning manager, wherein the analyzer interface module is configured to: receive a proposed run from the workflow planning manager, the proposed run comprising a plurality of requested analytical tests to be executed by the diagnostic analyzer on one or more biological samples, determine a candidate run schedule for executing the requested analytical tests in the proposed run according to an analyzer resource optimisation process, generate predicted run fulfilment information based on the candidate run schedule, the predicted run fulfilment information comprising an indication of which of the requested analytical tests in the proposed run can be processed by the analyzer in the candidate run schedule, and transmit the predicted fulfilment information to the workflow planning manager.

The system may comprise any of the features described above in respect of the fourth aspect.

In an eighth aspect of the present invention there is provided an analytical system for analyzing biological samples. The analytical system comprises: one or more diagnostic analyzers for performing an analysis on the biological samples, a transport device for transporting the biological samples to the one or more diagnostic analyzers, a workflow planning manager for coordinating the distribution of biological samples to the diagnostic analyzers, the workflow planning manager being communicatively connected to each diagnostic analyzer via an analyzer interface module and to the transport device. The workflow planning manager is configured to: receive a plurality of requests for analytical tests to be processed by the system, each analytical test corresponding to a biological sample; determine a proposed run comprising at least some of the requested analytical tests; transmit the proposed run to the analyzer interface module connected to a candidate diagnostic analyzer of the one or more diagnostic analyzers. The analyzer interface module is configured to: receive the proposed run from the workflow planning manager, determine a candidate run schedule for executing the requested analytical tests in the proposed run according to an analyzer resource optimisation process, generate the predicted run fulfilment information based on the candidate run schedule, the predicted run fulfilment information comprising an indication of which of the requested analytical tests in the proposed run can be processed by the analyzer in the candidate run schedule, and transmit the predicted fulfilment information to the workflow planning manager. The workflow planning manager is configured to: receive the predicted run fulfilment information from the analyzer interface module, and determine whether to proceed with or reject the proposed run based on the predicted fulfilment information, wherein: when the proposed run is to be proceeded with, the workflow planning manager is configured to signal the transport device to transport some or all of the biological samples specified in the proposed run to the candidate diagnostic analyzer. When the proposed run is rejected, the workflow planning manager may be configured to determine an alternative proposed run or transmit the proposed run to an alternative diagnostic analyzer device.

The system may comprise one or more (e.g., a plurality of) analyzer interface modules, each connected to one or more (e.g., respective) diagnostic analyzers and configured to operate as discussed above.

In an additional aspect, there is provided a computer-readable medium comprising instructions which when executed by a computer, cause the computer to execute the computer-implemented methods of the third and/or fifth aspects. Additional aspects of the invention may relate to systems configured to execute the computer-implemented methods discussed above. Specifically, the system may comprise a processor which is configured to execute the respective computer-implemented method aspects of the invention.

Additional aspects of the invention may provide a computer program comprising instructions which, when the program is executed by a computer, cause the computer to carry out the steps of a computer-implemented method of the above aspects of the present invention. Further aspects of the invention may provide a computer-readable storage medium, having stored thereon, the computer program of the previous aspects of the invention.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Fig. 1** shows a flow diagram of biological samples being processed in a molecular laboratory;
**Fig. 2** shows a flow diagram of an updated method for distributing and processing biological samples in the molecular laboratory;
**Fig. 3** shows a diagram of a system for processing biological samples according to aspects of the present invention;
**Fig. 4** shows a flow diagram of a process for coordinating the distribution of biological samples in an analytical system;
**Fig. 5** shows a flow diagram of a process for determining a proposed run for processing the biological samples;
**Fig. 6** shows a flow diagram of a process for negotiating and agreeing a proposed run;
**Fig. 7** shows a summary of a process for validating a proposed run;
**Fig. 8** shows a process for renegotiating a proposed run based on biological samples arriving at a diagnostic analyzer;
**Fig. 9** shows a flow diagram of a workflow planning manager agreeing a proposed run with a diagnostic analyzers;
**Fig. 10** shows a flow diagram of a process for interfacing between a diagnostic analyzer and a workflow planning manager;
**Fig. 11** shows a flow diagram of further process steps for interfacing between a diagnostic analyzer and a workflow planning manager;
**Figs. 12** - **15** show diagrams illustrating additional communications between a workflow planning manager, a diagnostic analyzer, and a transport device; and
**Fig. 16** shows a state diagram illustrating state transitions of an analyzer allocation slot.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

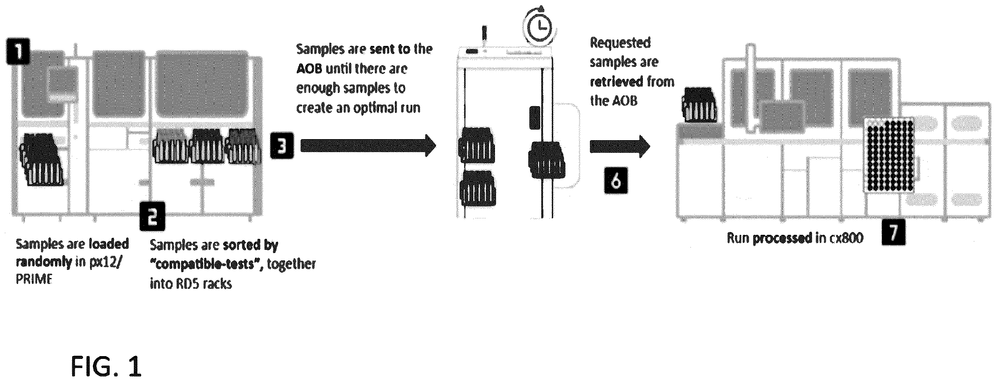

Fig. 1 shows a flow diagram of biological samples being processed in a molecular area of a laboratory.

First, at point 1, samples arrive in the laboratory in a continuous flow during working hours. The samples each have different characteristics including: types of tests to be performed on each biological sample such as Hepatitis B Blood (HBV) tests, human immunodeficiency virus (HIV) tests, etc; and target turnaround times (TAT). Additionally, each biological sample and corresponding test may be classified as an urgent or routine test.

In the laboratory, the samples might undergo different operations in a pre-analytics area (see point 2 of Fig. 1) before they are sorted in 5-position racks ("RD5 racks") in a buffer (AOB), to wait before being processed by one or more diagnostic analyzers (e.g., labelled "cx800").

Next, at point 6 the racks containing a samples for processing are retrieved from the AOB and transported to a diagnostic analyzer where the requested corresponding to those samples are processed at point 7.

The diagnostic analyzers, such as the cx800 shown in Fig. 1, work in runs, wherein each of the samples specified in a run are loaded into an analyzer before the entire run is executed. A run consists of, e.g., up to 96 requested tests to be processed in an analyzer. Each type of analyzer in a laboratory may have different capabilities, capacities, and throughput and so the time to process a run may vary. For example, a run may take between 2 to 3 hours.

Traditionally, each diagnostic analyzer may be provided with a predefined menu of tests to process. This menu is the result of a tactical decision which is made e.g., daily or weekly. Incompatibilities between tests exist and so two incompatible tests cannot be processed at the same time. The following table illustrates a possible test menu and configuration of a laboratory comprising three diagnostic analyzers (A1 - A3).

| Test name | Compatibility group | Analyzers allowed |
|---|---|---|
| HIV | A | A1,A2,A3 |
| HBV | A | A2 |
| CT-NG | A | A1 |
| MPLX | B | A2,A3 |

For example, the following operations may be possible using the analyzers in the table above:
A) if a run including HIV and HBV tests is sent to A2, the entire run can be processed;
B) if the same run including HIV and HBV texts is sent to A1, only HIV tests will be processed creating HBV samples as 'passenger samples'. In other words HBV test will be required to go back to the AOB, and must be included in another run;
C) if a run with HBV and MPLX tests is sent to A2, only the HBV tests or MPLX tests may be processed because of their compatibility groups also creating 'passenger samples'.

Accordingly, it is important to set up runs in an efficient manner to avoid unnecessary impacts on processing costs, turnaround times (TAT), resource usage, and the laboratory's compliance with regulatory standards (e.g., service level agreement (SLA) compliance). However, difficulties in predicting which test orders are going to arrive in the laboratory and when can detract from accurate planning throughout the day. Additionally, the creation and scheduling of non-full runs can decrease the overall occupation of the system thus increasing the costs per test order, costs and resource usage per test type, overall costs, and average throughput time. In further examples, passenger samples included in runs (i.e., samples which are sent to a diagnostic analyzer but cannot be processed by that analyzer) result in the need to create additional runs and additional sample transportation manoeuvres to process the passenger samples. Such passenger samples may overload the transportation system, create difficulties in setting up full runs, and put at risk the ability of the system to meet test target TATs. Further, the receipt of urgent samples can impact the whole system as they force the system to process them immediately, to the detriment of efficient operation e.g., by causing delays to previously planned runs. Accordingly, the composition of the runs, capabilities of the transportation system, and the amount of time in which the analyzers need to process a run can each impact the overall performance of the system. Furthermore, since only the diagnostic analyzers themselves have access to all the information about their status (e.g., including reagent status, expected time to finish a run, etc) it is challenging to determine a laboratory workflow which makes efficient use of the resources available in the laboratory.

Traditional methods for coordinating a laboratory may assess test compatibilities (as demonstrated in the above table) and then apply a simple algorithm such as FIFO, Greedy etc to determine a workflow. However the present inventors have found that such methods are inadequate for ensuring efficient operation of the laboratory.

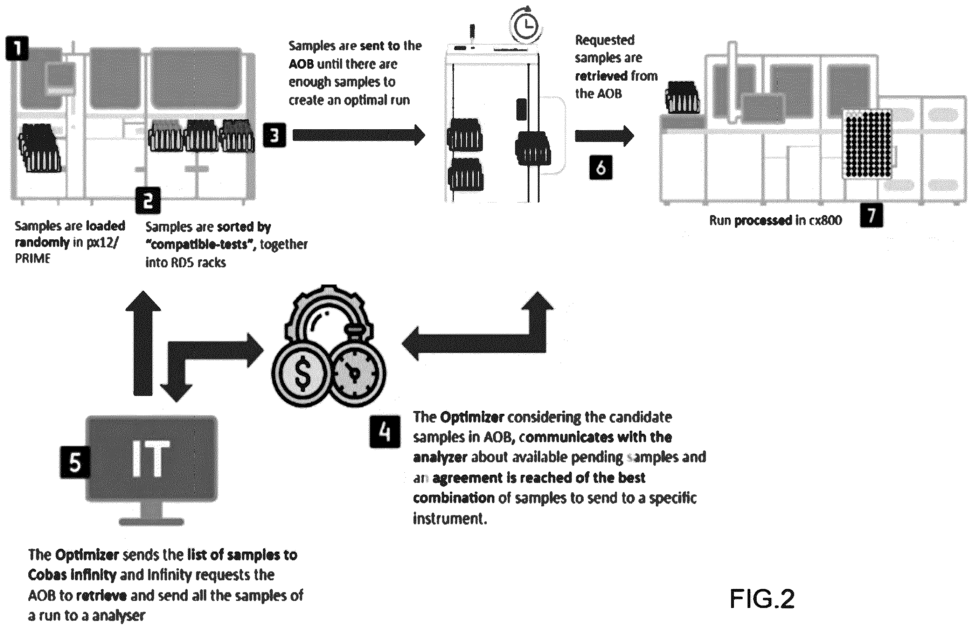

Fig. 2 shows a flow diagram of an updated method for distributing and processing biological samples in a molecular laboratory. In addition to the steps of Fig. 1, an optimizer is introduced in Fig. 2 which is configured to perform an intermediate process comprising: considering the samples in the buffer (e.g., unprocessed samples which have arrived in the laboratory), communicating with the diagnostic analyzer, and reaching an agreement about an optimised possible combination of samples to send to the analyzer as a run. The agreed run is then sent to the diagnostic analyzer which then requests the samples specified in the run from the buffer and processes the samples according to the requested tests in the agreed run.

In this way, more efficient use of the analyzers in the laboratory can be achieved whilst enabling the system to react more effectively to incoming samples and changes in analyzer statuses, e.g., as resources get used up. In particular, this approach is advantageously able to accommodate and react to specific, continuously varying and unforeseeable constraints of the instruments in the laboratory (e.g., the diagnostic analyzers, or transport device). For example, such constraints may include transportation time, processing and transport bottle-necks, barcode errors, analyzer processing capacity, availability of materials and consumables etc. By using a reactive approach to determining the workflow as described herein, only currently available resources are scheduled for use, whilst ensuring that the schedule is as flexible as possible so as to react to new (and potentially urgent) samples and test orders that may arrive in the laboratory.

The following figures provide more detail on the method for determining the workflow and distribution of the biological samples in this way.

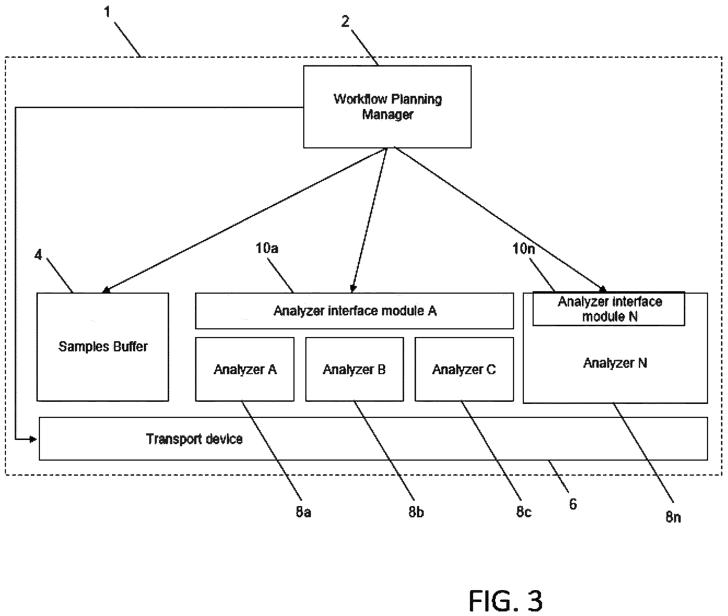

Fig. 3 shows a diagram of an analytical system 1 for analyzing biological samples. For example, system 1 may be a laboratory as shown in Fig. 2.

The system 1 comprises one or more diagnostic analyzers 8n for performing an analysis on the biological samples, a transport device 6 (e.g., transportation system) for transporting the biological samples to the one or more diagnostic analyzers 8n, and a workflow planning manager 2 for coordinating the distribution of biological samples to the diagnostic analyzers 8n.

The workflow planning manager 2 is communicatively connected to each diagnostic analyzer 8n and to the transport device 6. The workflow planning manager 2 (e.g., coordinator, planning component, planning module, central controller, etc) is configured to oversee all devices in the system 1 (e.g., including Pre/Post-Analytic devices, diagnostic analyzers 8n, the transport device 6, etc) as well as all the sample tubes that are available at any time in the system 1. As discussed herein, the workflow planning manager 2 is configured to execute planning algorithms, based on constraint-programming, as well as forecasting models that, based on historical data collected from the laboratory, are used to calculate an optimised allocation of samples to the diagnostic analyzers 8n at a given point in time referred to as a workflow.

In this example, the system 1 comprises a plurality of diagnostic analyzers 8a-n and the workflow planning manager 2 is communicatively connected to each diagnostic analyzer 8n via one or more analyzer interface modules 10n which are each configured to provide an interface between one or more of the diagnostic analyzers 8n and the workflow planning manager 2. As shown in Fig. 3, each analyzer interface module 10n may be connected to one or more diagnostic analyzers 8n. Alternatively, each analyzer interface module 10n may form part of a diagnostic analyzer 8n, e.g., as a software application running on the diagnostic analyzers 8n. In further examples, each analyzer interface module 10n may be a separate component to the diagnostic analyzer 8n, for example, as software running on a central server and connected to one or more of the diagnostic analyzers 8n.

Herein, the term "module" is used to refer to a functional module which is configured or adapted to execute a particular function. The modules may be implemented in hardware (i.e. they may be separate physical components within a computer), in software (i.e. they may represent separate sections of code, which when executed by a processor, cause the processor to perform a particular function), or in a combination of both.

The workflow planning manager 2 is configured to receive a plurality of requests for analytical tests (e.g., test orders) to be processed by the system 1, each analytical test corresponding to a biological sample. Incoming biological samples are stored in racks in a sample buffer 4 until they are transported to one of the diagnostic analyzers 8n by the transport device 6.

The workflow planning manager 2 is configured to negotiate with one or more of the diagnostic analyzers 8n to agree a workflow comprising one or more runs of the requested tests and then direct the transport device 6 to transport the biological samples to the diagnostic analyzers 8n in order to process the agreed runs. Each diagnostic analyzer 8n is configured to perform local optimization of proposed runs based on loaded samples, consumables, reagents, and other resources which are present in the analyzer 8n so as to generate a locally optimised run schedule. The run may then be executed (or not) according to the locally optimised run schedule.

Importantly, the workflow planning manager 2 is configured to continuously renegotiate and redetermine the planned runs as additional samples and test orders arrive in the system 1 or as statuses of the diagnostic analyzers 8n change. Accordingly, the system 1 is reactive and adaptable to changing situations and able to determine more efficient workflows for processing the test orders than existing solutions, without needing prior knowledge of which test orders are going to be requested ahead of time.

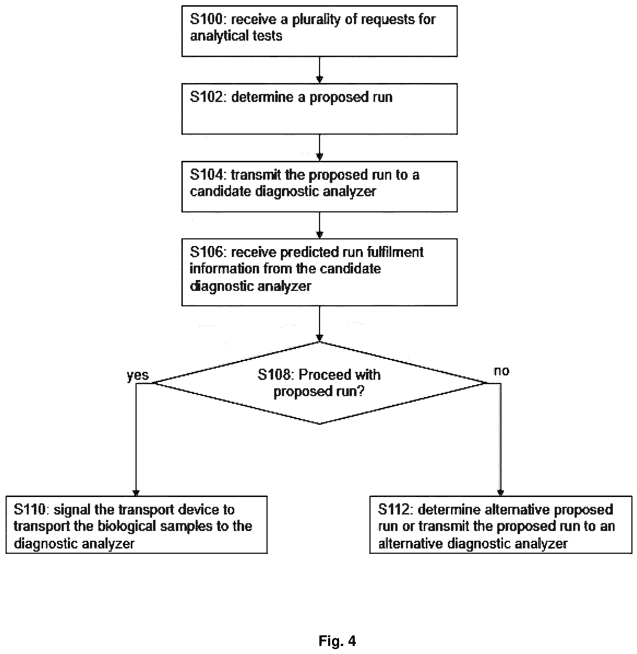

Fig. 4 shows a flow diagram of a process for coordinating the distribution of biological samples in the system 1. The process may be performed by the workflow planning manager 2 of Fig. 2. The following steps may be separated into two stages comprising a planning stage wherein a proposed run is prepared based on information available in the system 1, and then an execution stage wherein the workflow planning manager 2 negotiates the proposed run with the analyzers 8n and manages any unforeseeable events that may occur up until execution of the proposed run is started.

First, in step S100, the workflow planning manager 2 receives a plurality of requests for analytical tests. The requests may be referred to as test orders, each test order comprising an identifier for a biological sample, a specification of one or more processes to perform on the biological sample, and, optionally, a target TAT for the test order.

Next, in step S102 the workflow planning manager 2 determines a proposed run comprising at least some of the requested analytical tests. The proposed run is determined by selecting a plurality of tests from the requested tests according to a workflow optimisation scheme. The workflow optimisation scheme is a process of selecting tests for the proposed run so as to meet the target TATs and/or group compatible tests together.

In addition to the requested tests, the workflow planning manager 2 may also have access to information including a number of tests per test type which have been received, the target TAT for each test, and compatibility information indicating which tests are compatible with each other and may be executed in the same run by a same diagnostic analyzer 8n. Additionally, the workflow planning manager 2 has access to a current status of each analyzer 8n, and a menu of tests that each analyzer 8n is able to execute. The workflow planning manager 2 can therefore use this information to construct the proposed run and to select one or more candidate diagnostic analyzers 8n to send the proposed run to.

Every time a change in the system 1 is detected such as: a new sample arriving in the sample buffer 4, the priority of a test order changing, test orders being added to or removed from a sample, or a change in the status of one or the diagnostic analyzers 8n (e.g., on/off, run starts, run ends, etc) the workflow planning manager 2 is configured to retrieve the above mentioned information and determine whether a new proposed run may be constructed based on the updated information. This action may also be performed periodically, for example, after a predetermined number of minutes has elapsed since the last time a proposed run was determined.

Next, in step S104, the proposed run is transmitted to a candidate diagnostic analyzer 8n of the one or more diagnostic analyzers 8n (e.g., by transmitting the proposed run to an interface module 10n of the candidate diagnostic analyzer 8n).

Next, in step S106, the candidate diagnostic analyzer 8n receives predicted run fulfilment information from the candidate diagnostic analyzer 8n. The predicted run fulfilment information comprises an indication of which of the requested analytical tests in the proposed run can be processed by the candidate diagnostic analyzer 8n. For example, the predicted run fulfilment information may take the form of a counter offer (e.g., a counter proposal, an additional/alternative proposed run, a modified proposed run).

Next, in step S108, the workflow planning manager 2 reviews the predicted run fulfilment information and determines whether to proceed with or reject the proposed run based on the predicted run fulfilment information and the results of an optimisation checking process. Proceeding with the proposed run may therefore comprise accepting the proposed run, as modified if necessary, in view of the predicted run fulfilment information.

Determining whether to proceed with or reject the proposed run according to the optimization checking process comprises comparing the predicted run fulfilment information to predetermined acceptance criteria such as: whether the run is full enough (e.g., by determining if a threshold percentage of the proposed run can be fulfilled by the candidate diagnostic analyzer 8n); whether there are samples and/or requested tests in the proposed run indicated as urgent, and if those samples and/or requested tests can be fulfilled by the candidate diagnostic analyzer; and/or whether an estimated time in which the run can start is soon enough to meet the target TATs of the requested tests in the proposed run. If each of the (or one or more of the) acceptance criteria are satisfied, then the workflow planning manager 2 is configured to proceed with the proposed run. However, if one or more of, or all, of the acceptance criteria are not satisfied, then the workflow planning manager 2 is configured to reject the proposed run.

When the proposed run is to be proceeded with, the workflow planning manager 2 proceeds to step S110 where the workflow planning manager 2 signals the transport device 6 to transport some or all of the biological samples specified in the proposed run to the candidate diagnostic analyzer. For example, the transport device 6 may be instructed to transport each of the biological samples specified in the proposed run to the candidate diagnostic analyzer 8n, or just the biological samples which are specified predicted run fulfilment data (e.g., the biological samples specified in the proposed run which the candidate diagnostic analyzer 8n has indicated it can process).

Notably, the diagnostic analyzer 8n will not execute the proposed run until it receives all of the samples specified in the agreed run or a confirmation order from the workflow planning manager 2. In this way, if an unexpected event occurs when sending the samples to the candidate analyzer 8n (such as not all of the samples arrived on time owing to bottle-necks or barcode reading errors) then the workflow planning manager 2 can update the workflow by determining whether to execute the run immediately with the samples that did arrive, discard the run, or wait for the samples to arrive before executing the run.

When the proposed run is rejected, the workflow planning manager 2 proceeds to step S112 where it determines an alternative proposed run (comprising a different selection of requested tests) or transmits the proposed run to an alternative candidate diagnostic analyzer 8n. Determining an alternative proposed run may be viewed as returning to the beginning and repeating the process of Fig. 4.

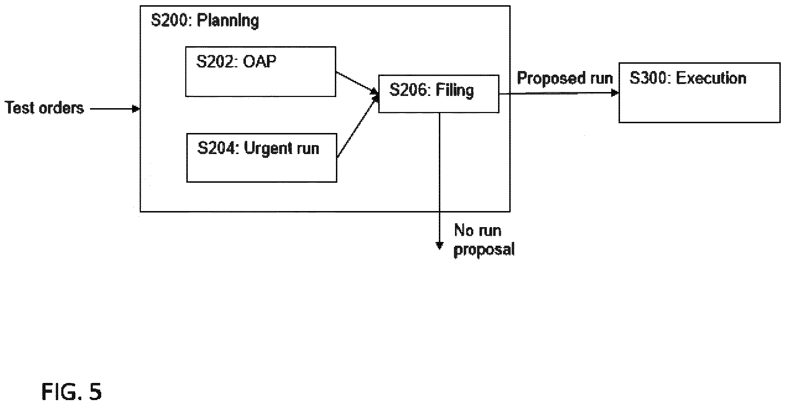

Fig. 5 shows a flow diagram of a process for determining the proposed run according to the workflow optimization scheme. As mentioned above, this process may be referred to as a planning stage S200, which is performed prior to the execution stage S300. The workflow optimization scheme comprises three algorithms for selecting requested tests to populate the proposed run with. The three algorithms are executed according to the following flow:

In step, S302, an OaP (Override and Process) selection algorithm is used to select test orders for populating one or more proposed runs. This algorithm is executed only where there is the need to execute one or more types of test in the next immediate runs which is triggered by a specific action from a user, e.g., when the laboratory is at an End-of-Shift. The selection of the test is being done with its TAT and in case two or more tests exist with the same TAT but one of them is indicated as being urgent, the latter will be picked first.

When an OaP state is triggered (e.g., by a user) the OaP selection algorithm is used to populate one or more runs until a set of test orders indicated by the user are processed. The system will remain in OaP until all of that set of test orders are processed, in a more immediate run, as per the user request. Accordingly, a session wherein the OaP selection algorithm is used is not limited to a single run, but a number of runs that are necessary to process the user test type selected for OaP processing.

If there are not enough samples to set up a full run then a filling algorithm in step S206 (explained below) will be executed in an attempt to fill the run. Alternatively, if the proposed run is full but there are still samples impacted by OaP, then the proposed run will be transmitted to a candidate analyzer and the OaP selection algorithm will remain activated and the workflow planning manager 2 will continuously create additional proposed runs until the OaP state (which was triggered by the user) is finished.

Alternatively, if the OaP selection algorithm is not activated by a user, then the workflow planning manager will determine the proposed run according to an urgent run selection algorithm in step S204.

The aim of the urgent selection algorithm is to set up a run comprising requested tests which need to be executed immediately otherwise their target TATs will be compromised. This algorithm is a mathematical optimization algorithm based on Mixed-Integer Programming (MIP). The algorithm picks only the tests that fall into a predefined planning horizon (e.g., 4 hours). That is, requested tests whose associated target TAT is included in the planning horizon. The urgent selection algorithm may be used to determine a plurality of proposed runs to accommodate each of the requested tests whose target TAT falls into the planning horizon, each run being allocated to a candidate analyzer 8n, and a time when each run should be executed so as to meet the target TATs of the tests in the run.

Additionally, the MIP algorithm of the urgent selection algorithm can optionally be used to determine a selected analyzer for executing the proposed run.

The urgent run selection algorithm is configured to delay the execution of each proposed run as much as possible while still meeting the target TATs of the tests in the run. The advantage of this is to gain visibility about additional requests for tests and biological samples arriving in the system 1 (e.g., in the buffer). Owing to the reactive operation of the workflow planning manager this means that more pending tests may be received and so a likelihood of being able to create full and optimized runs is increased.

After populating the proposed run according to the OaP and/or urgent run selection algorithms, the workflow planning manager 2 is configured to proceed to step S206 wherein the proposed run is populated with additional requested tests, according to a filling algorithm, in order to fill the run. If the run is already full, then the workflow planning manager 2 will proceed directly to the execution stage S300 where the proposed run is negotiated with one or more of the diagnostic analyzers 8n before being executed or rejected.

The filling algorithm can be triggered subsequent to the execution of the OaP algorithm or the urgent run algorithm or independently irrespective of whether the previous algorithms proposed a run. For example, if OaP is not triggered by a user and there are no imminently due requested tests (i.e., urgent tests), then the workflow planning manager may proceed directly to the filling algorithm for determining the proposed run.

The filling algorithm is configured to update a run proposed by the previous algorithms to be as full as possible or, if there is no existing run proposal, attempt to create a full run comprising only filler tests (e.g., requested tests for which the target TAT is not at risk). The filling algorithm is configured to select tests according to the following rules, listed in order of priority:
i. Passenger samples minimization: passenger samples are the samples that are included in a rack that will be sent to an analyzer 8n for processing tests but where no test is to be executed for these specific samples. Such samples will visit the analyzer 8n and then, after the run is executed, will be transported back to the buffer 4. The time spent by such samples in the analyzer 8n is therefore inoperative time because the samples cannot be allocated to another run until they are returned to the buffer. Accordingly, the workflow planning manager is configured to populate each proposed run with requested tests whose samples are located a same rack which is going to be sent to the analyzer 8n thus reducing the presence of passenger samples.
ii. Requested tests which are due soon: the potential tests to be included in a run are sorted and allocated to the proposed run according to their associated target TAT, for example, from soonest to furthest due.
iii. Tie-break: next, tests may be used to populate a proposed run according to a time of arrival of their respective biological samples in the system 1 (e.g., according to arrival time in the buffer 4).

In addition, each of the selection algorithms of steps S202-206 may each also account for one or more of the following factors:
- STAT (Short Turn Around Time) samples: Corresponds to those that need to be run urgently and therefore have a lower shorter) TAT than other tests of the same type. The algorithms focus on meeting the TAT but in case of conflict where the algorithms need to choose between two samples with the same remaining TAT but one of them is indicated as STAT (e.g., urgent), then the latter will be selected first.
- Test compatibility: the workflow planning manager 2 (implementing any of the above selection algorithms) is configured to take into consideration that there are types of test which cannot be executed together in the same run.

Prioritisation when tests are already due: if a requested test is already due wherein its target TAT has already been exceeded, then the workflow planning manager 2 (implementing any of the above selection algorithms) is configured to prioritise the selection of tests which have been due for the longest. For example, one test has been due for two hours and three tests have been due for one hour, the workflow planning manager 1 is configured to select the test which has been due for two hours first.

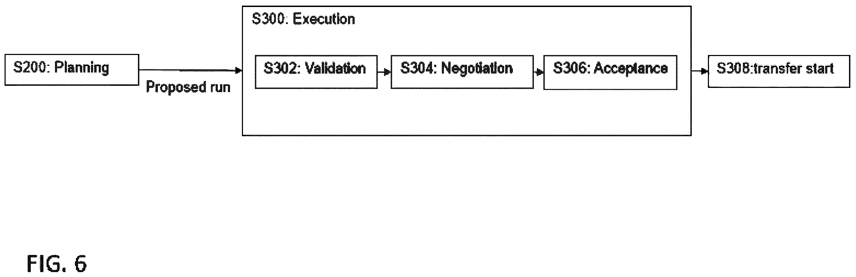

Fig. 6 shows a flow diagram of a process for validating and agreeing the proposed run between the workflow planning manager 2 and diagnostic analyzers 8n, and for determining whether to proceed with the proposed run or not. This part of the process may be referred to as the execution stage S300 as mentioned above.

First, in step S302, the workflow planning manager 2 is configured to validate the proposed run to determine if it is suitable, prior to negotiation with the candidate diagnostic analyzer 8n.

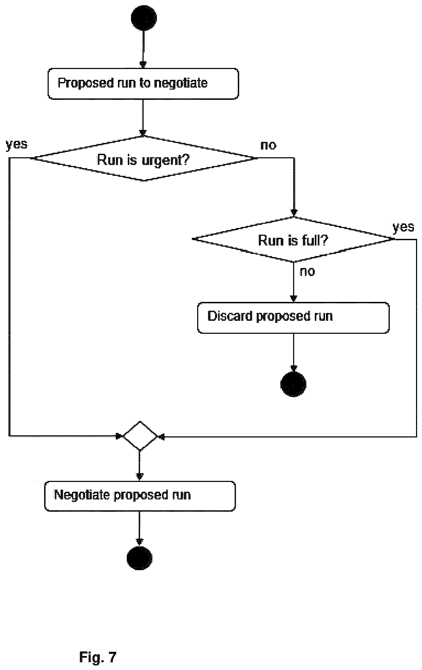

Fig. 7 shows a summary of a process for validating the proposed run. As shown in Fig. 7 Validating the proposed run involves determining if the proposed run is urgent or if the proposed run is full.

An urgent run may be a run which contains tests that must be executed now, otherwise their target TAT won't be met. For example, urgent runs may be proposed runs which have been output by the urgent run selection algorithm discussed above for Fig. 5.

Determining if a proposed run is full may comprise comparing the number of requested tests and/or samples in the proposed run to a target fill number. The target fill number may be a maximum capacity of the run or a certain percentage of the maximum capacity, the maximum capacity being based on e.g., capabilities of the system 1, rack sizes, and the diagnostic analyzers 8n. For example, the maximum samples that a run contains may be 96, and a run may be considered full if it contains at least 96 - x samples, where x may be e.g., between 5 and 20 samples.

The target fill number may be relaxed (i.e., reduced) at the end of a shift, e.g., prior to a scheduled laboratory down time. At this time, new samples and requested tests may cease to arrive and so the workflow planning manager 2 is able to stop prioritising the scheduling of full runs, since this may not be possible at the end of the shift.

When the proposed run is determined to be full or urgent the proposed run is successfully validated and can be negotiated with an analyzer 8n. However, when the proposed run is determined to be neither full nor urgent, the proposed run is discarded. By accepting unfilled runs only if they are urgent, the laboratory can be operated more efficiently. For example, an un-urgent run can be delayed until enough test orders come in to fill it, or the run may be recomputed to contain more test orders.

Returning to Fig. 6, when the proposed run is successfully validated, the workflow planning manager 2 proceeds to step S304 to negotiate the validated proposed run with one or more of the diagnostic analyzers 8n. The negotiations include transmitting the proposed run to a candidate diagnostic analyzer 8n for assessment and, in response, receiving predicted run fulfilment information from the candidate analyzer 8n as discussed above for steps S1 04-S1 06 of Fig. 4.

To the negotiate the proposed run, the workflow planning manager 2 is configured to, first, select one of the diagnostic analyzers 8n as the candidate analyzer 8n. If the proposed run was an output of the urgent run algorithm, then the analyzer 8n which the run needs to be executed on may be known and is selected as the candidate analyzer 8n. Otherwise, for example, if the proposed run was an output of the filling algorithm, the workflow planning manager 2 will look for a most suitable of diagnostic analyzers 8n available. In this example, the workflow planning manager 2 will assess all of the available diagnostic analyzers 8n that can accept a run, and look for those that have the requisite reagents installed to execute the proposed run. From those, the workflow planning manager 2 is configured to select the diagnostic analyzer 8n which has been idle for the longest as the candidate analyzer 8n. The purpose of this is for balancing the load of the diagnostic analyzers 8n in the system 1.

After selecting the candidate analyzer 8n, the workflow planning manager 2 is configured to transmit the proposed run to the candidate analyzer 8n, e.g., via an interface module 10n of the candidate analyzer 8n. The candidate analyzer 8n is then configured to perform a local optimisation process to decide whether each of the tests in the proposed run can be executed or not based on different factors such as:
1) if there is sufficient reagent available;
2) if additional QC (quality control) steps and samples are required to execute the proposed run; QC's are included by the instrument in a run occupying one or more positions out of the e.g., 96 positions that a run may have. Generally a QC may be required once every 24 hours for each type of test, although this requirement may be adjusted by a user. The workflow planning manager does not have knowledge of whether a QC is going to be required in a run. For example, the workflow planning manager may propose a run containing 96 test orders and samples. The candidate analyzer 8n may counter offer to perform only 94 of the proposed tests because e.g., two of the positions in the run must be assigned to QCs.
3) Ensuring that analyzer specific requirements are followed by the different types of tests in the proposed run; For example, the number of tests per type of test a run may need to be performed in pairs. Therefore,, if a proposed run comprises five positions assigned to a type of test, then six positions in the run may be reserved by the candidate analyzer for this test type.
4) If each of the tests in the proposed run are compatible for execution in the same diagnostical analyzer 8n.

If the candidate diagnostic analyzer 8n cannot execute all the requested tests included in the proposed run, then the candidate diagnostic analyzer 8n will also take into account the priorities of each requested test (e.g., their target TAT, with soonest having the highest priority). The candidate diagnostic analyzer 8n then sends a counter proposal (e.g., in the form of predicted run fulfilment information) to the workflow planning manager 2 which includes an indication of the requested tests that can be processed, the requested tests that cannot be processed, the reasons for that, and a predicted run start time indicating when the candidate analyzer 8n may next be available to execute the proposed run.

Next, the workflow planning manager 2 proceeds to step S306 to determine whether to accept (e.g., proceed with) the proposed run based on the predicted run fulfilment information from the candidate analyzer 8n.

To determine whether to proceed with the proposed run, the workflow planning manager 2 is configured to determine, from the predicted run fulfilment information, if a threshold percentage of requested tests in the proposed run can be executed by the candidate analyzer 8n. For example, the threshold may be 90% of the proposed tests. Additionally, the workflow planning manager 2 is configured to determine if the predicted run start date and/or time is within a threshold deviation of an expected start date and/or time which was determined by the workflow planning manager 2 when it determined the proposed run. If the above conditions are met, then the workflow planning manager 2 is configured to proceed to step S308 and signal the transport device 6 to begin transferring the required biological samples to the candidate analyzer 8n to execute the proposed run.

However, if the workflow planning manager 2 determines that the predicted run fulfilment information is too different from the initial proposed run, for example, because one or more of the above-mentioned thresholds is not met, then further action is required. The further action that the workflow planning manager 2 might take depends on the root cause of the unfulfillment by the candidate analyzer 8n. For example, the following root causes and further actions may be determined:

Cause: reagent or QC's locked or missing: this might be because the candidate analyzer 8n (which may be able to execute two runs in parallel) has locked a reagent cassette needed for a specific test in the proposed run in order to perform a different run that it is already executing. In other examples, there may be missing QC material in the candidate analyzer so certain tests cannot be executed.

Action 1) If one or more tests affected are urgent, the workflow planning manager 2 will record that the test type is not available in that analyzer 8n and will execute the planning again by redetermining an alternative proposed run and/or by selecting an alternative candidate analyzer.

Action 2) If no urgent tests are affected then the workflow planning manager 2 will remove the requested test(s) which cannot be performed from the proposed run and will run the filling selection algorithm again so as to generate a new proposed run.

Cause: The candidate diagnostic analyzer 8n is unavailable or a capacity of the analyzer 8n has decreased.

Action: In this case, the workflow planning manager 2 is configured to exclude this analyzer 8n from the list of available analyzers 8n and then redetermine the proposed run and reselect a candidate analyzer 8n.

Cause: A reagent cassette change is required.

Action: In this case, the workflow planning manager 2 is configured to accept and proceed with the proposed run.

When the workflow planning manager 2 decides to proceed with the proposed run, it instructs the transport device 6 to transfer the samples to the candidate diagnostic analyzer 8n. Next, racks containing the samples begin to arrive at the candidate analyzer 8n. If all of the racks arrive at the analyzer on time (e.g., by a reserved allocation slot of the analyzer 8n), then the workflow planning manager 2 signals the analyzer to being processing the proposed run. However, if one or more of the racks didn't arrive at the analyzer 8n on time then the workflow planning manager 2 is configured to determine whether to delay, proceed with, or discard the proposed run.

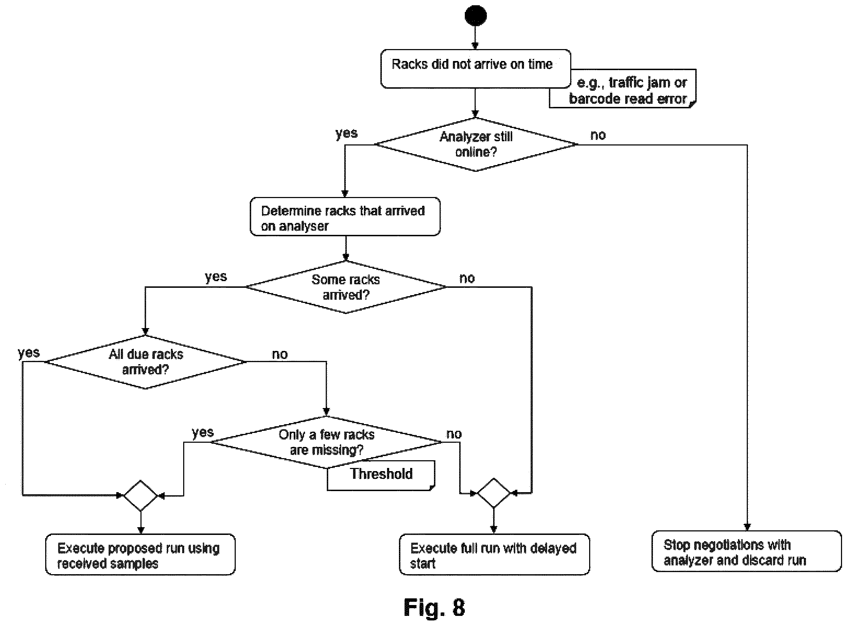

Fig. 8 shows a process for monitoring biological samples arriving at the candidate diagnostic analyzer 8n and renegotiating a proposed run if samples are not received at the candidate diagnostic analyzer 8n. The samples arrive at the candidate diagnostic analyzer candidate diagnostic in racks, each rack being configured to contain a plurality of biological samples. However, if one or more of the expected racks are missing e.g., owing to a barcode reading error or a traffic jam in the transport system 6, then the analyzer interface module 10n sends a message to the workflow planning manager 2 which performs the evaluation of Fig. 8.

First, if all racks that contained biological samples corresponding to tests marked as urgent arrived at the candidate diagnostic analyzer 8n then the workflow planning manager 2 is configured to instruct the candidate diagnostic analyzer 8n to execute the run. Otherwise if the number of racks pending to arrive is fewer than a threshold number of racks (for example 2 racks) then it is assumed that there is a barcode error and manual user intervention is required. In this case, the run is renegotiated and executed with the samples that did arrive at the candidate diagnostic analyzer 8n.

However, if more than the threshold number of racks has arrived at the candidate diagnostic analyzer 8n, then the cause of the missing racks is identified as a delay caused by a traffic jam. In this case, the workflow planning manager 2 renegotiates the same run with the candidate diagnostic analyzer 8n but with a delayed start for when the racks have arrived. This renegotiation will only happen once. Therefore, if the one or more samples/racks are still missing at the candidate diagnostic analyzer 8n at the time of the renegotiated (delayed) run, then the run will not be renegotiated again and is discarded.

Once the workflow planning manager 2 has signalled the proposed run to begin, it continues to monitor the system 1 for events, such as new test orders being received, and then redetermines and negotiates new proposed runs as needed.

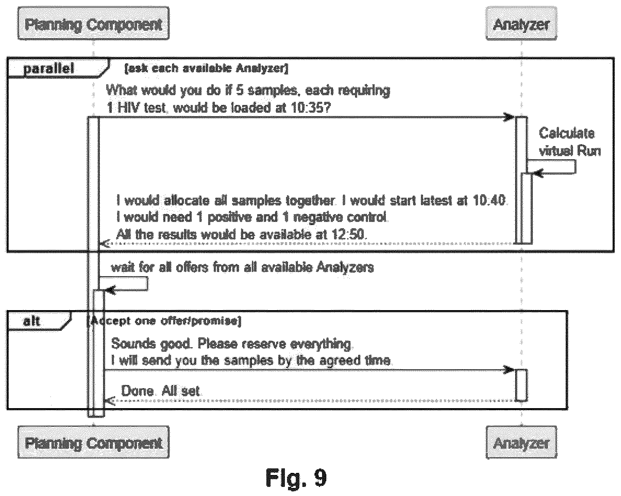

As discussed above, the system 1 may comprise an analyzer interface module 10n configured to handle negotiations and communications between the workflow planning manager 2 and one or more of the diagnostic analyzers 8n. The interface between workflow planning manager 2 and each diagnostic analyzer 8n is operated based on the theory of promises [2]. The following description of Figs. 9 to 16 is directed to this interface.

The workflow planning manager 2 is the only component within the system 1 that has access to a status of each diagnostic analyzer 8n, and transport device 6 within the system 1. Further, the workflow planning manager 2 is the only component in the system 1 that is aware of all the biological samples which are currently available. On the other hand, each diagnostic analyzer 8n only knows (and cares) about its internal state and status, thus applying Demeter's law [1] whereby an object of a system only interacts with its immediate neighbours and has limited knowledge of other objects' inner workings. This means that each diagnostic analyzer 8n only cares about loaded biological samples (physically loaded within that analyzer 8n) and work orders associated with those biological samples. The diagnostic analyzers 8n are not configured to perform any processing or planning to account for biological samples which are not within their scope (e.g., physically loaded into that analyzer 8n).

Accordingly, the interface module 10n operates to reconcile the information held by each component in the system 1 so as to agree a proposed run to process the samples.

Fig. 9 shows a diagram of communications being exchanged between the workflow planning manager 2 (labelled "planning component") and a diagnostic analyzer 8n. Fig. 9 illustrates the general approach of the interaction between these two components in pseudo-language.

The interaction starts by the workflow planning manager 2 asking each diagnostic analyzer 8n how and when they would schedule a proposed run if a specific set of biological samples were to be loaded (e.g., 'virtually loaded samples') in the diagnostic analyzer 8n before or at a given point in time. The analyzers 8n are then responsible for executing their internal logic and assessing their internal state, which includes knowledge about loaded consumables and resources as well as the availability of hardware-redundant components to propose an allocation for each of the 'virtually loaded samples' into a 'virtual run' (e.g., a candidate run schedule).

Next, each of the diagnostic analyzers 8n respond to the workflow planning manager 2 with a 'promise' (e.g., a counter offer) which provides the workflow planning manager 2 with enough information so that the promise or offer can be evaluated against offers from each of the other diagnostic analyzers 8n. For example, the promise may be in the form of predicted run fulfilment information described above.

The workflow planning manager 2 may be configured to prioritise one or more optimization goals as discussed above (e.g., to reduce costs, e.g., QC-related costs), to increase throughput, or to reduce total TAT of all of the requested tests in the system 1. Using the optimization goals, together with historical data and forecasting models, the workflow planning manager 2 can then select the best promise (e.g., counteroffer) from the diagnostic analyzers for fulfilling the optimization goal (e.g., according to the above mentioned optimization checking scheme).

If one promise is determined to be acceptable then the workflow planning manager 2 informs the corresponding analyzer 8n that its promise has been accepted. At this point, the analyzer 8n must reserve and allocate the necessary resources (e.g., reagent cassettes) so that, once the agreed biological samples are loaded into the selected diagnostic analyzer 8n, the promise can be immediately fulfilled. As mentioned above, if the proposed run is rejected in view of the promises received from each analyzer 8n, then the workflow planning manager 2 may begin new negotiations with the diagnostic analyzers 8n using an alternative proposed run.

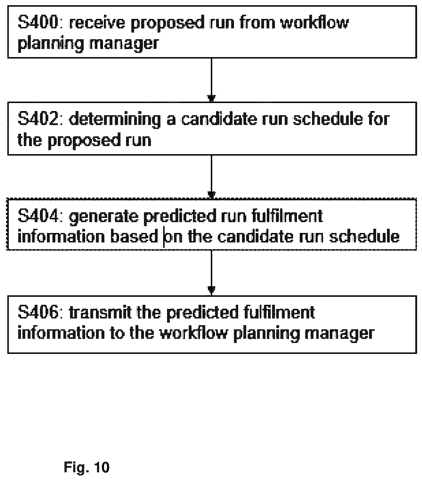

Fig. 10 shows a flow diagram of the interaction process between a diagnostic analyzer 8n and the workflow planning manager 2. This process may be performed by one or more of the interface modules 10n of Fig. 3. As discussed above, each interface module 10n may form part a diagnostic analyzer 8n.

First, in step S400, the interface module 10n receives the proposed run from the workflow planning manager 2 as part of a request for a proposed allocation slot. The proposed run comprises a plurality of requested analytical tests to be executed by a diagnostic analyzer 8n.

Next, in step S402, the interface module 10n determines a candidate run schedule according to an analyzer 8n resource optimization process. The analyzer 8n resource optimization process is a local scheduling function performed by the candidate diagnostic analyzer 8n to determine a candidate schedule for processing a set of test orders. The analyzer 8n resource optimization process may therefore be conducted by proprietary software of that particular diagnostic analyzer 8n, based on the specific resources, settings, and capabilities of that analyzer 8n.

Next, in step S404, the interface module 10n generates (or receives) predicted run fulfilment information based on the candidate run schedule (e.g., a promise or counter offer). The predicted run fulfilment information comprises, at least, an indication of which of the requested analytical tests in the proposed run can be processed by the analyzer 8n according to the candidate run schedule. The predicted run fulfilment information may also comprise information about the proposed allocation slot of the analyzer 8n for when the proposed run can next be executed according to the candidate run schedule. For example, the proposed allocation slot may be a period of time when the diagnostic analyzer 8n will next become available and/or when the diagnostic analyzer 8n next expects to have the required resources available to perform the proposed run.

Finally, in step S406, the interface module 10n transmits the predicted run fulfilment information to the workflow planning manager 2 to determine if the proposed run should be proceeded with in view of the predicted run fulfilment information.

If the workflow planning manager 2 rejects the proposed run (e.g., based on the predicted run fulfilment information or because new test orders have been received triggering the proposed run to be recalculated) or if the workflow planning manager 2 has chosen to proceed with the proposed run using another diagnostic analyzer 8n, then the process may end there until another proposed run is received.

However, if the workflow planning manager 2 determines that the proposed run should be proceeded with, then the interface module 10n is configured to reserve the proposed allocation slot of the analyzer 8n for the proposed run and arrange for the diagnostic analyzer 8n to perform the proposed run in that slot.

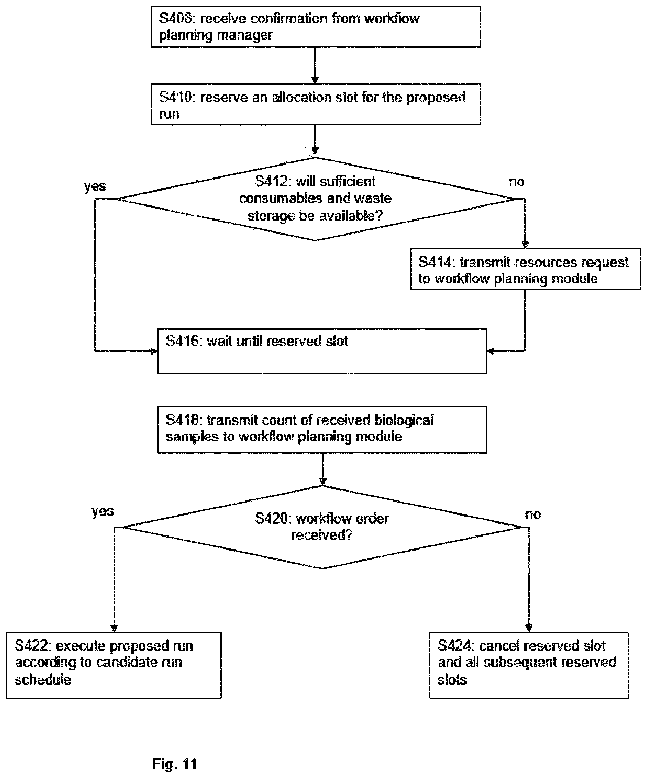

Fig. 11 shows a flow diagram of further process steps for interfacing between a diagnostic analyzer 8n and the workflow planning manager 2 when the proposed run is proceeded with. The process of Fig. 11 may therefore be performed subsequent to the process of Fig. 10. As in Fig. 10, the process of Fig. 11 may be performed in part, or entirely, by an interfacing module running middleware and communicating with the diagnostic analyzer 8n, or by the diagnostic analyzer 8n itself.

First, in step S408, the interface module 10n (or the diagnostic analyzer 8n) receives confirmation from the workflow planning manager 2 that the proposed run has been accepted in view of the predicted run fulfilment information. The confirmation may take the form of a request to reserve an allocation slot of the analyzer 8n for executing the proposed run. The interface module 10n then reserves (or signals the diagnostic analyzer 8n to reserve) an allocation slot for the proposed run. For example, the interface module 10n may reserve the proposed allocation slot which was determined and proposed to the workflow planning manager 2 earlier.

The allocation of a slot to a proposed run is governed by the following rules. Firstly, each analyzer 8n can received multiple requests for allocation slots before one of the proposed slots is then accepted by the workflow planning manager 2. The analyzer 8n will not consider previously determined but unaccepted allocation slots when making a new one but it will consider previously accepted but not yet realized allocation slots. Accordingly, the analyzer 8n is configured to avoid scheduling pending runs in a same slot so as to avoid a clash.

Further, when each diagnostic analyzer 8n determines and reserves an allocation slot for the proposed run, it is configured to account for one or more of the following factors:
- upcoming planned maintenance windows of the analyzer 8n;
- reduced performance due to unavailability of hardware-redundant modules on the analyzer 8n;
- which reagent cassettes are presently loaded in the analyzer 8n;
- how many control mini-racks are presently loaded in the analyzer 8n.

However, when the diagnostic analyzer 8n assigns and reserves an allocation slot, it will NOT consider:
- availability of consumables in the analyzer 8n; or
- capacity of waste storage in the analyzer 8n.

Rather, the diagnostic analyzer 8n is configured to inform the workflow planning manager 2 of missing resources required to execute the next run (e.g., a proposed run for which a next allocation slot is reserved). If the needed resources are not loaded by a scheduled start time of an agreed run, the reserved allocation slot is cancelled.

In addition, the following rules governing allocation slots are applied. First, an allocation slot is considered realized as soon as the execution of a proposed and accepted run has begun. Additionally, the diagnostic analyzer 8n is configured to begin an allocation slot (and process the planned run assigned to that slot) as soon as all the agreed samples of the planned run have been received by the analyzer 8n, even if this happens before an agreed start-time of the allocation slot (assuming that all resources, supplies and needed consumables are available at the analyzer 8n). When the workflow planning manager 2 requests an allocation slot, the diagnostic analyzer 8n (or interface module) is configured to assign and reserve an allocation slot for only the next (and only one) possible run. An allocation slot will never be assigned to (e.g., be reserved for) more than one proposed run.

Once an allocation slot has been reserved for the proposed run, the process of Fig. 10 proceeds to step S412, where the diagnostic analyzer 8n (and/or interface module 10n) determines if a current stock of consumables in the diagnostic analyzer 8n is sufficient, and/or if enough waste storage is available in the diagnostic analyzer 8n, to perform the proposed run in the reserved allocation slot. If the consumables are sufficiently stocked and there is sufficient waste storage available, then the process proceeds to step S416. However, if more consumables or waste storage is needed then, the process proceeds to step S414, where a signal is transmitted to the workflow planning manager 2 requesting that the consumables be restocked and/or that the waste storage is emptied.

In step S416, the interface module 10n waits until a start time of the reserved allocation slot. While waiting, the diagnostic analyzer 8n may be performing a different run which was previously scheduled, performing a maintenance routine, or be in an idle state. During this waiting period, the transport device 6 is operated to transport the biological samples specified in the proposed run to the diagnostic analyzer 8n. The diagnostic analyzer 8n registers and takes a count of biological samples arriving at and being loaded into the diagnostic analyzer 8n.

Next, in step S418, at a start time of the reserved allocation slot, the interface module 10n transmits the count of received biological samples to workflow planning module. The interface module 10n may also transmit ID numbers of the received biological samples.

The workflow planning module 2 then determines whether to proceed (or not) based on the count of received biological samples as discussed above. For example, if all of the biological samples have arrived, then the workflow planning manager 2 may proceed. However, if biological samples are missing, e.g., owing to bottlenecks in the system 1, bar code reading errors, hardware failure etc, then the workflow planning manager 2 may determine, based on the count and which biological samples have arrived, whether to cancel or delay the reserved allocation slot or whether to proceed with the proposed run in the reserved slot using only the received biological samples.

Accordingly, in step S420 the interface module 10n checks to see whether a workflow order has been received from the workflow planning manager 2 confirming that the proposed run should begin.

If a workflow order has been received, then the interface module 10n proceeds to step S422 and signals the diagnostic analyzer 8n to execute the proposed run (e.g., according to the candidate run schedule) using the received biological samples. However, if a workflow order has not been received (e.g., by a predetermined time limit after a start time of the reserved slot), or if a cancellation order is received instead, then the interface module 10n proceeds to step S424 and cancels the reserved allocation slot. All subsequent reserved slots which have already been allocated for a time period after the reserved allocation slot are also cancelled so that they can be renegotiated. The reasoning for this is that subsequent allocation slots will have been calculated under the premise that the current allocation slot would be realized. If this is not the case, assumptions made in their calculation may be broken and so the present inventors have found that it is more efficient redetermine all proposed runs and allocated slots based on the actual resources that are available and all of the unprocessed test orders.

Additional behaviour rules followed by the analyzer interfacing module 10n include:
- If there is a sample barcode reading error, the diagnostic analyzer 8n will load the rack anyway. The allocation slot may get revoked because a work order was not received from the planning module on time at which point the rack can be unloaded again.
- If no connection to the workflow planning module 2 is available before a workflow order has been received, then accepted allocation slots cannot be kept, and the scheduled runs for those slots are not executed. However, if work orders have been received from the workflow planning manager 2 (e.g., after all of the biological samples corresponding to a run have arrived at the diagnostic analyzer 8n) before the connection to the workflow planning module 2 is lost then the run is executed.
- If a rack is received or loaded onto an analyzer 8n wherein none of the biological samples within that rack are part of any allocation slot (e.g., required to be processed in a scheduled run) then the rack will be immediately unloaded and returned to the buffer 4. However, an exception to this rule is if one or more of the samples in the rack were scheduled to be used in a run that was not executed (e.g., part of a not-fulfilled allocation slot) then the rack is held for a period of time (e.g., 5 minutes).

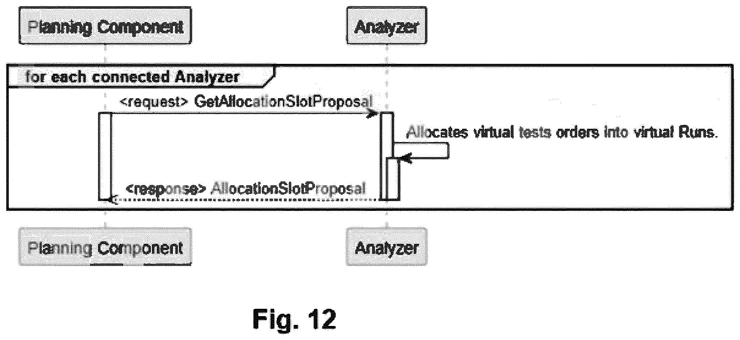

Figures 12 to 15 are flow diagrams showing communications between the workflow planning manager 2, a diagnostic analyzer 8n, and the transport device 6. These figures show examples of the specific commands and data transmitted between the workflow planning manager 2 and the diagnostic analyzers 8n during the above described interactions.

Fig. 12 shows a diagram depicting the workflow planning manager 2 requesting an allocation slot for performing a proposed run (i.e., GetAllocationSlotProposal) and a diagnostic analyzer 8n responding with a proposed allocation slot. The workflow planning manager 2 is configured to request each available diagnostic analyzer 8n (which is capable of processing the specific tests in a proposed run) for an allocation slot. Each diagnostic analyzer 8n will then execute internal logic to consider loaded reagent cassettes, availability of hardware redundant modules, and predetermined control rules to determine a candidate run schedule including a proposed allocation slot defining a time period for performing the proposed run. The proposed allocation slot is transmitted to the workflow planning manager 2 (i.e., AllocationSlotProposal), for example, as part of the predicted run fulfilment information discussed above.

The GetAllocationSlotProposal message contains a list of Sample Containers, and for each sample container: a sample container ID (e.g., a barcode), a list of test(s) to be executed, a test code, a test priority status, and a latest estimated arrival time of the sample container at the diagnostic analyzer 8n.

The AllocationSlotProposal message may contain:
- an allocation slot Identifier,
- an allocation slot expiration time (indicating how long the allocation slot will be valid for until it expires if it is not accepted or declined by the workflow planning manager),
- a sample group including: a list of sample test order identifiers allocated into the sample group, a list of newly added controls (only if new controls have to be used), an estimated time for when processing can be started, and a delta time indicating when results can be made available

a list of non-scheduled sample test orders including sample test order identifiers and a reason for why each identified sample test order was not scheduled in the candidate run schedule.

The allocation slot proposal (e.g., the predicted run fulfilment information) is not stored persistently on the diagnostic analyzer 8n. Therefore, only the workflow planning module 2 has knowledge of each different allocation slot proposal from each of the diagnostic analyzers 8n and will select the most suitable of the proposed slots for executing the proposed run in. As mentioned above, if none of the proposed allocation slots are determined to be suitable, the workflow planning manager 2 is configured to request new proposals from the analyzer 8n(s).

However, if the workflow planning manager 2 decides to accept the proposed allocation slot and proceed with the proposed run, then it will reserve the proposed allocation slot by sending a command to the relevant analyzer 8n.

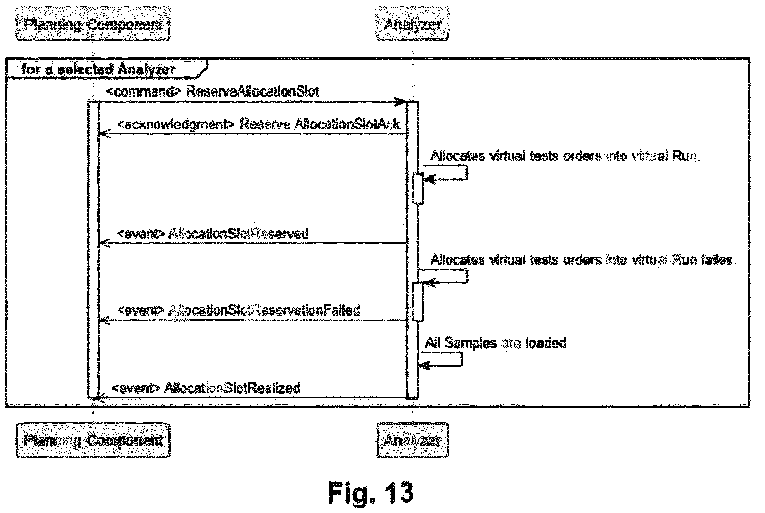

Fig. 13 shows a flow diagram of the communications involved in reserving a proposed allocation slot.

First, the workflow planning manager 2 sends a ReserveAllocationSlot command containing the following data:
- Target Instrument (e.g., the candidate diagnostic analyzer 8n than proposed the accepted allocation slot)
- Proposed allocation slot identifier

List of orders containing, for each sample container: a sample ID (e.g., a barcode); test information including test(s) to be executed on that sample container, a test code (e.g., USI); test priority status; a latest predicted arrival time; and a latest run start time

Next, the diagnostic analyzer 8n respond with an acknowledgement message (ReserveAllocationSlotAck) followed by an indication that the allocation slot has been reserved (AllocationSlotReserved Event message) containing: the target instrument ID, an allocation slot identifier, and an allocation slot status. Next, the diagnostic analyzer sends an indication to the workflow planning manager 2 indicating whether or not the reservation of the allocation slot was successful or not (e.g., AllocationSlotRealization Event or AllocationSlotReservationFail Event).

At a certain point in time, the workflow planning manager operates the transport device 6 to transport the biological samples (organized in RD5 racks) to the diagnostic analyzer 8n that reserved the allocation slot. As discussed above, in relation to Fig. 8, the diagnostic analyzer 8n is configured to inform the workflow planning manager 2 about the arrival of the biological samples and which biological samples have not yet arrived at the analyzer 8n. If one or more of the samples belonging to a reserved allocation slot does not arrive at the diagnostic analyzer 8n at the specified latest arrival time, then the diagnostic analyzer 8n is configured to discard the reserved allocation slot and all subsequent reserved allocation slots as well. This puts the responsibility back to the workflow planning manager 2 to decide what to do next with the samples, as some of them may have arrived at the analyzer 8n already. The workflow planning manager 2 is configured to determine whether to renegotiate or delay the proposed run depending on which samples have arrived at the analyzer 8n as discussed above, in relation to Fig. 8.

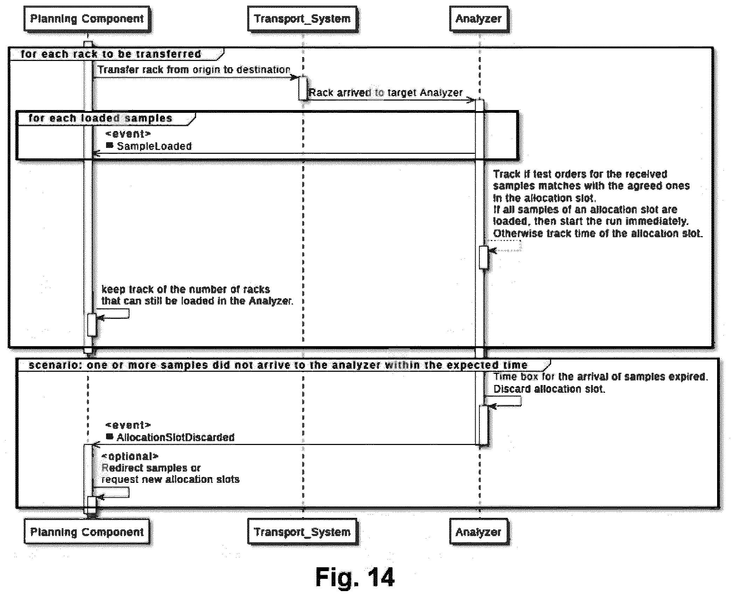

Fig. 14 shows a sequence diagram of this interaction.

First a SampleLoaded event is sent from the analyzer 8n to the workflow planning manager 2 comprising a sample container ID (e.g., sample tube barcode). Notably, if all of the samples required to be processed in a reserved allocation slot have arrived, the diagnostic analyzer 8n will execute the proposed run. However, if one or more samples are missing, then the diagnostic analyzer 8n will wait until the scheduled start time of the reserved allocation slot to pass before sending an indication to the workflow planning manager 2 that the reserved allocation slot is to be discarded (AllocationSlotDiscarded message). At this point the workflow planning manager 2 must determine how to proceed according to the method of Fig. 8.

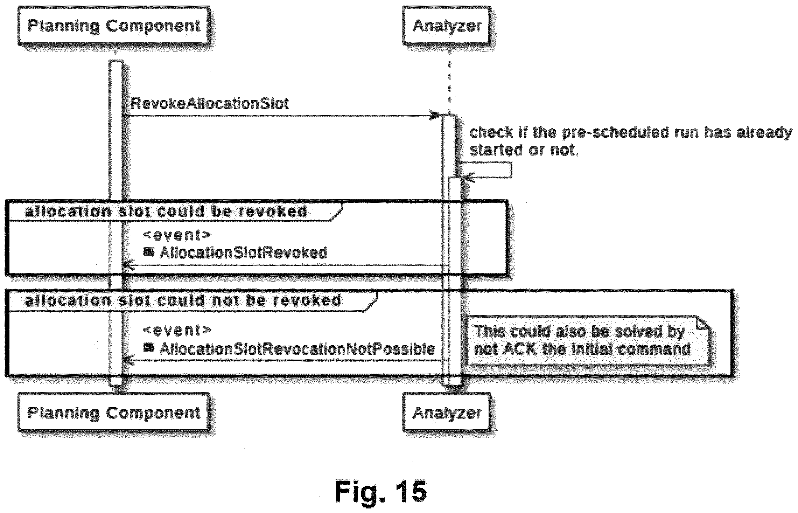

In some examples, the workflow planning component may wish to cancel a proposed run and revoke a reserved allocation slot associated with that proposed run (for example to renegotiate the proposed run in view of changes in the system and/or new urgent test orders arriving in the system). This interaction is illustrated in Fig. 15.

To cancel a reserved allocation slot, the workflow planning manager 2, first, sends a command ("RevokeAllocationSlot") to the diagnostic analyzer 8n instructing the diagnostic analyzer 8n to cancel (revoke) the reserved allocation slot.

If execution of the proposed run scheduled for that allocation slot has not yet started, then the diagnostic analyzer 8n cancels the allocation slot and responds to the workflow planning manager 2 with an AllocationSlotRevoked message containing an allocation slot ID. However, if execution of the proposed run in the reserved allocation slot has already started, then the diagnostic analyzer 8n will respond with an AllocationSlotRevocationNotPossible message which contains the Allocation Slot ID and a reason for not revoking the allocation slot.

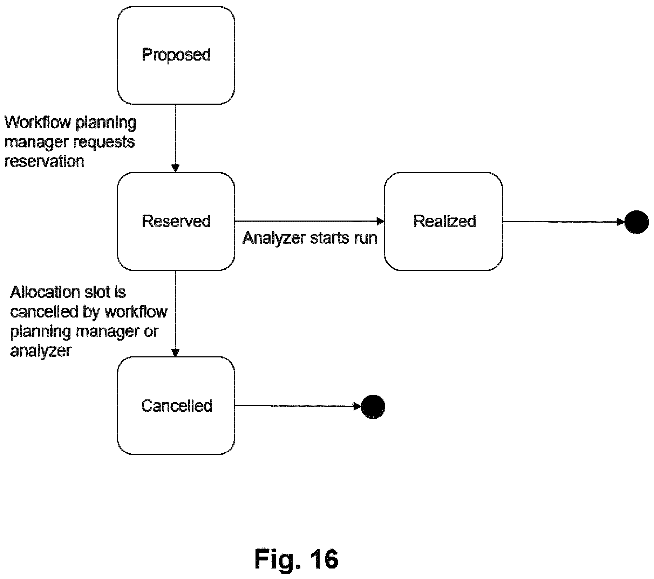

Fig. 16 shows a state diagram illustrating the different states of an allocation slot as managed by a diagnostic analyzer 8n. These states are summarised in the following table.

| **State** | **Description** |
|---|---|
| Proposed | An allocation slot proposal has been created by the analyzer 8n and sent back to workflow planning manager |
| Reserved | An allocation slot in the proposed state which has been reserved by the workflow planning manager |
| Cancelled | An allocation slot in the reserved state which was either revoked by the workflow planning manager 2 or discarded by the analyzer 8n (e.g., if expected samples have not arrived at the system) |
| Realized | The analyzer 8n has started executing the test orders scheduled in the reserved allocation slot. This allocation slot may now not be revoked by the workflow planning manager 2. |

The following table summarizes transitions between each of the states and the triggers which may lead to each transition. These triggers and transitions are managed locally by the diagnostic analyzer 8n (or analyzer interface module 10n).

| **Transition** | **Trigger** | **Description** |
|---|---|---|
| Proposed -> Reserved | ReserveAllocationSlot | after evaluating all the received allocation slots proposals the workflow planning manager may choose one of them and then then send a reservation request to one of the analyzers 8n to reserve the proposed allocation slot from that analyzer 8n |
| Reserved -> Cancelled | The workflow planning manager asks to cancel a reserved allocation slot or the analyzer 8n decides to discard the allocation slot itself | The workflow planning manager may determine that a previously reserved allocation slot should be revoked because it no longer lead to an optimised workflow. Alternatively, the analyzer 8n may discard a previously reserved allocation slot, for example, if expected samples have not arrived at the analyzer 8n by the agreed time |
| Reserved -> Realized | Analyzer 8n started processing the biological samples that belong to an allocation slot | When all samples have arrived on time at the analyzer 8n, the analyzer 8n is configured to immediately start processing them. |

By employing the methods described herein, a laboratory can be operated more efficiently without requiring that the workflow planning manager 2 has access to all information about the capabilities and present states of each diagnostic analyzer 8n in the system or the current load of each analyzer 8n. Advantageously, this collaborative approach enables efficient operation without needing to expose the internal operations of each component in the system 1 to each other thereby fulfilling Demeter's law, the benefits of which include increased maintainability and increased adaptability of the system 1 (e.g., the laboratory) and the components within in. Since each component is less dependent on the internals of the others, it is easier to change the internal operations of each component without impacting other components in the system 1.

Further, a benefit or the proposed solution compared to known solutions is the fact that the optimization schemes performed by the workflow planning manager 2 and the local optimization algorithms in the analyzers 8n can be changed and evolved independently. Changing one does not require changing the other.

In this way, the proposed solution enabled the decoupling of the workflow planning manager 2 and the analyzer 8n thus providing all the benefits of a modular solution when it comes to evolvability, maintainability and performance.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A workflow planning manager for coordinating the distribution of biological samples in an analytical system, the analytical system comprising:
one or more diagnostic analyzers for performing one or more tests on the biological samples, and a transport device for transporting the biological samples to the one or more diagnostic analyzers,
wherein the workflow planning manager is configured to:
receive a plurality of requests for analytical tests to be processed by the system, each analytical test corresponding to a biological sample,
determine a proposed run comprising at least some of the requested analytical tests;
transmit the proposed run to a candidate diagnostic analyzer of the one or more diagnostic analyzers,
receive predicted run fulfilment information from the candidate diagnostic analyzer, the predicted run fulfilment information comprising an indication of which of the requested analytical tests in the proposed run can be processed by the candidate diagnostic analyzer, and
determine whether to proceed with or to reject the proposed run based on the predicted run fulfilment information, wherein:
when the proposed run is to be proceeded with, the workflow planning manager is configured to signal the transport device to transport some or all of the biological samples specified in the proposed run to the candidate diagnostic analyzer, and
when the proposed run is rejected, the workflow planning manager is configured to determine an alternative proposed run or transmit the proposed run to an alternative candidate diagnostic analyzer.

2. The workflow planning manager according to claims 1 wherein determining the proposed run comprises:
selecting a plurality of tests from the requested tests according to a workflow optimization scheme, and
populating the proposed run with the selected tests.

3. The workflow planning manager according to claim 2 wherein selecting the plurality of tests according to the workflow optimization scheme comprises selecting the tests based on:
a number of tests per type of test in the requested tests,
a target turn-around-time of each requested test, and/or
compatibilities between the requested tests; so as to select requested tests for the proposed run which:
are able to be processed in a same diagnostic analyzer,
have compatible target turn-around-times,
are tests of a same type of test, and/or
minimises a number of passenger samples in the proposed run.

4. The workflow planning manager according to any of claims 2 or 3 wherein selecting the plurality of tests according to the workflow optimization scheme comprises:
first, selecting requested tests which are indicated as urgent, and
then, selecting tests having the soonest target turn-around-times of the requested tests.

5. The workflow planning manager according to any of claims 2 to 4, wherein determining the proposed run comprises:
selecting an initial plurality of tests according to the workflow optimization scheme, and
then filling the proposed run by allocating additional tests to the proposed run to meet a target fill number.

6. The workflow planning manager according to any preceding claim wherein the workflow planning manager is further configured to:
determine a maximum delay which can be applied while still meeting target turn-around-times associated with each of the requested tests in the proposed run, and
wait for the maximum delay before transmitting the proposed run to the candidate diagnostic analyzer.

7. The workflow planning manager according to any preceding claim wherein:
the workflow planning manager is configured to validate the proposed run prior to transmission to the candidate diagnostic analyzer by determining if the proposed run is full and/or if the proposed run is urgent, wherein:
when the proposed run is determined to be full or urgent, the proposed run is transmitted to the candidate diagnostic analyzer, and
when the proposed run is determined to be neither full nor urgent, the proposed run is discarded.

8. The workflow planning manager according to any preceding claim further configured to:
detect a system change and
responsive to detecting the system change, determine the proposed run;
wherein the system change may be detected in response to one or more of:
a new biological sample arriving in the system,
a requested test being added or removed from the plurality of requested tests, or
an operating status of one of the diagnostic analyzers being updated.

9. The workflow planning manager according to claim 8 further configured to: repeatedly determine new proposed runs in response to detecting a system change until each of the requested tests in the plurality of requested tests is executed.

10. The workflow planning manager according to any preceding claim,
wherein the workflow planning manager is configured to transmit the proposed run to a plurality of candidate diagnostic analyzers and receive respective predicted run fulfilment information from each of the plurality of candidate diagnostic analyzers,
wherein determining whether to proceed with or reject the proposed run comprises analyzing the respective predicted fulfilment information from each diagnostic analyzer, and
wherein proceeding with the proposed run comprises selecting, based on the respective predicted run fulfilment information, one of the candidate diagnostic analyzers for executing the proposed run.

11. The workflow planning manager according to any preceding claim, wherein determining whether to proceed with or reject the proposed run comprises:
comparing the predicted run fulfilment information to acceptance criteria, the acceptance criteria comprising one or more of:
a threshold percentage of requested tests in the proposed run which can be fulfilled by the candidate diagnostic analyzer,
whether there are requested tests in the proposed run indicated as urgent, and if those requested tests indicated as urgent can be fulfilled by the candidate diagnostic analyzer, and/or
a target time for beginning the proposed run.

12. The workflow planning manager according to any preceding claim wherein, when the proposed run is to be proceeded with, the workflow planning manager is further configured to:
receive a transport update from the candidate diagnostic analyzer indicating which of the biological samples have arrived at the candidate diagnostic analyzer, and
transmit a signal to the diagnostic analyzer indicating whether to execute the proposed run, discard the proposed run, modify the proposed run, or delay the proposed run based on the transport update.

13. The workflow planning manager according to any preceding claim further configured to:
detect an end of shift condition indicating that no further requested tests are incoming; and
responsive to detecting the end of shift condition, reduce acceptance criteria for determining whether to proceed with or reject the proposed run and/or lower a target fill number for filling the proposed run.

14. A computer-implemented method for coordinating an analytical system for analyzing biological samples, the system comprising:
one or more diagnostic analyzers for performing an analysis, each diagnostic analyzer comprising an analyzer programming module for operating the diagnostic analyzer;
a transport device for transporting the biological samples to the one or more diagnostic analyzers, and
a workflow planning manager for performing the computer-implemented method, the computer-implemented method comprising:
receiving a plurality of requests for analytical tests to be processed by the system, each analytical test corresponding to a biological sample,
determining a proposed run comprising at least some of the requested analytical tests,
transmitting the proposed run to a candidate diagnostic analyzer of the one or more diagnostic analyzers,
receiving predicted run fulfilment information from the analyzer programming module of the candidate diagnostic analyzer, the predicted run fulfilment information comprising an indication of which of the requested analytical tests in the proposed run can be processed by the candidate diagnostic analyzer,
determining whether to proceed with or reject the proposed run based on the predicted run fulfilment information, and
when the proposed run is to be proceeded with, signalling the transport device to transport some or all of the biological samples specified in the proposed run to the candidate diagnostic analyzer, and
when the proposed run is rejected, determining an alternative proposed run or transmitting the proposed run to an alternative diagnostic analyzer.

15. A computer-readable medium comprising instructions which when executed by a computer, cause the computer to execute the computer-implemented method of claim 14.
